# EUROPEAN PATENT APPLICATION

(11) **EP 3 272 366 A2**
(43) Date of publication of application: **24.01.2018**
(21) Application number: 17182221.6
(22) Date of filing: 14.02.2011
(51) Int. Cl.: A61L 9/013, A61L 9/16, B01D 46/00, D06M 15/01

(54) **METHODS AND APPARATUS FOR COMBATING SICK BUILDING SYNDROME**

(30) Priority: 15.02.2010 US 705843; 22.03.2010 US 316110 P; 04.06.2010 US 351390 P
(62) Divisional of application: 11704396.8
(71) Applicant: Philadelphia University, Philadelphia, PA 19144 (US)
(72) Inventor: Messinger, Alexander A., Ardmore, PA 19003 (US); Cundell, Diana R., Philadelphia, PA 19128 (US); George, Brian R., Pottstown, PA 19464 (US); Dhamankar, Bhalchandra, Newark, Delaware 19702 (US); Shumilova, Ekaterina, Perkasie, PA 18944 (US)
(74) Representative: Stafford, Jonathan Alan Lewis

(57) **Abstract**

Methods and apparatus for combating sick building syndrome include a plenum that is at least partially bounded by fabric comprising at least one naturally occurring botanically based, antimicrobial, morbidity-inducing fabric and preferably a fan for introducing air into the plenum for passage outwardly through the fabric thereby to cleanse the air of microbes contributing to sick building syndrome.

## Description

### Background of the Invention - Field of the Invention

This invention relates to maintenance and improvements of internal environments within buildings and more specifically relates to methods and apparatus for combating "Sick Building Syndrome" ("SBS").

### Background of the Invention - Description of the Sick Building Syndrome Problem and Prior Art Approaches to the Problem

Current design of buildings seeks to maximize energy efficiency and comfort for the building inhabitants using centralized heating and cooling systems. As a result, buildings now being built are becoming increasingly airtight, relative to buildings of prior years. Combined with the use of inexpensive building materials such as particle board, drywall and acoustical tile used as ceiling tiles, the modern design and construction approach has fostered a series of ailments affecting people living and working in these building environments. These ailments have been collectively termed Sick Building Syndrome (SBS).

Sick buildings are characterized by poor air circulation and imbalance in humidity which together allow build-up of biological and chemical contaminants.

The adverse impact, both economically and on public health, is significant: The United States Environmental Protection Agency has estimated that $61 billion dollars are lost in medical costs and worker absenteeism annually.

It is further estimated that if ventilation and lighting were improved in commercial buildings in the United States, there would be somewhere between 16 million and 37 million fewer cases of influenza or the common cold each year, and an 8% to 25% decrease in symptoms for the 53 million persons that suffer from allergies and the 16 million persons who are asthmatic in the United States, and further that there would be a 20% to 50% reduction in so-called "Sick Building Syndrome Health Symptoms".

SBS health symptoms have been reported to be most prevalent in persons suffering from allergies and asthmatics, whose sensitivity is often greatest to even low levels of indoor airborne biological contaminants that include microbes, especially molds. Since allergies are estimated to affect at least 1 person in every 6 of the population of the United States, build-up of these indoor contaminants is clearly of great concern.

While studies have shown that locations with SBS may have high levels of both airborne molds and bacteria, most researchers have devoted their efforts to the study of the molds and their effects due to the ease of identification of molds, the dramatic levels of spore release and responsiveness of molds to remediation by increasing air flow and decreasing humidity. Spore release by molds can amount to as high as approximately 300,000 spores per gram of mold mass.

The current consensus among environmental microbiologists is that elevated levels of at least 3 genera of airborne molds, namely penicillium, aspergillus and alternaria, can produce symptoms of SBS. These species, together with cladosporium, are believed to constitute greater than 90% of the viable mold flora in ambient air in a variety of environments, with up to a 50% increase in airborne alternaria and cladosporium occurring in Fall and Winter.

Elevated levels of airborne staphylococci as well as aerosolized water contaminated by legionella or gram-negative bacteria and their products have also been linked with SBS. Collectively, these bacteria comprise the dominant species in ambient air and are important agents for a wide range of infectious respiratory, gastro-intestinal and cutaneous human diseases.

Products currently on the market and promoted to remove such airborne contaminants are primarily focused against allergens and work to trap them in electrostatically-charged filters, which require periodic replacement or cleaning.

### Summary of the Invention

In one of its aspects, this invention provides a series of unique modular, adaptable and esthetically pleasing units for retrofitting tightly sealed buildings to combat Sick Building Syndrome (SBS). The units utilize fabric to capture airborne molds and bacteria responsible for SBS and, in some embodiments, also work to diffuse and balance indoor light levels. Measured reductions in indoor mold levels from ambient air using modular units according to the invention within a one month operational period ranged from 71% to 83%.

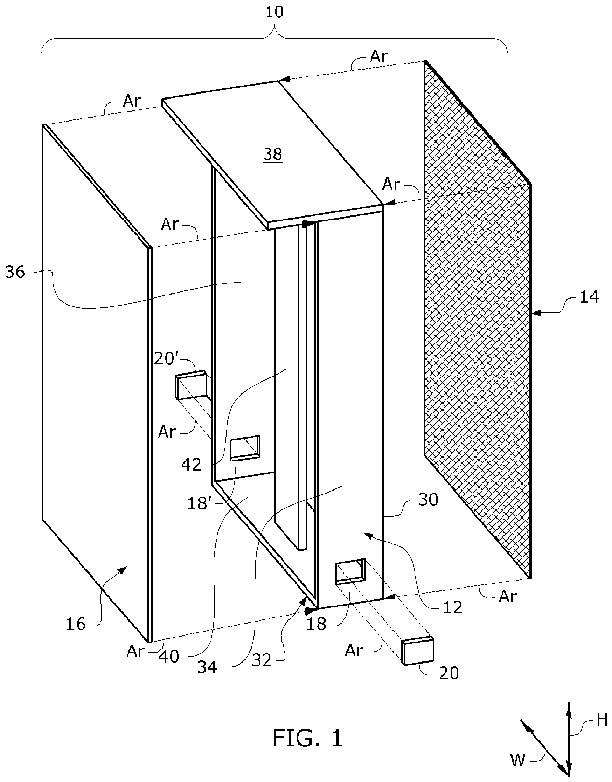

In another one of its aspects, as illustrated generally in Figure 1, this invention provides a preferably modular unit for improving indoor air quality where the unit includes a frame surrounding an open interior and defining an outer periphery of the unit, air permeable, preferably naturally occurring botanically based antimicrobial, morbidity-inducing fabric secured to the frame periphery on a first side of the frame and covering the open interior of the first side of the frame, an air impermeable member secured about the frame periphery on the remaining side of the frame and covering the frame periphery on the remaining side, at least one aperture preferably formed in the frame and being adapted to house a fan therein with an optional second aperture preferably being provided for a second fan or for air bleed, and at least one fan housed in the aperture for blowing air from outside the frame into the frame interior, for subsequent passage of blown into the frame interior outwardly through the fabric.

The fabric is preferably secured to the frame by Velcro; the air impermeable member is preferably fiberboard; the frame preferably has four members, two of which are preferably spaced apart lateral members, with the remaining two being bottom and top members; the unit preferably further includes a bracing member extending between and affixed to both the bottom and top members; the frame is preferably rectangular.

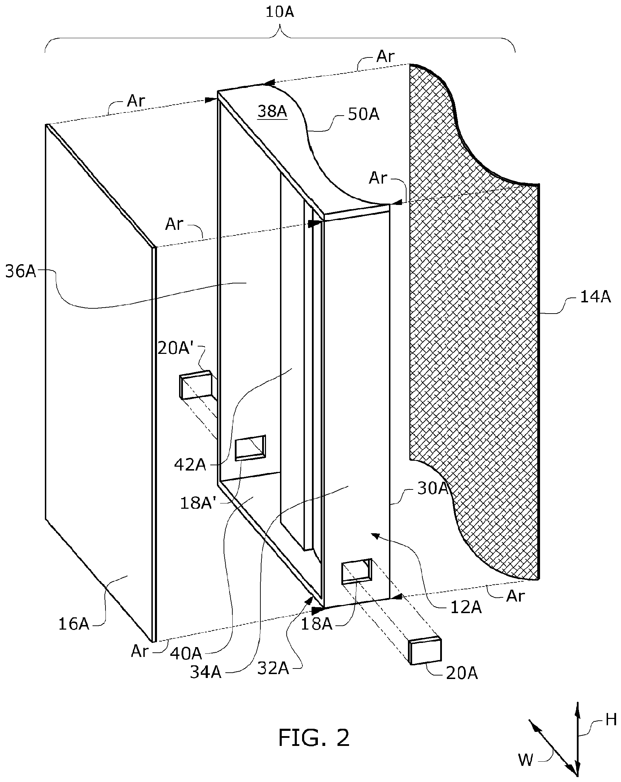

In the unit illustrated generally in Figure 2, the unit bottom and top members have edges preferably defining top and bottom portions of the first side of the frame periphery that are curved; the curved edges are preferably in the form of an S-curve; the curved edges of the top and bottom members preferably are tangent to the upstanding members at juncture therewith, and further preferably the edges of the top and bottom members are parallel. The fabric may be curved along the vertical length of the fabric in the same shape as the edges of the top and bottom portions of the first side of the frame periphery. The edges of the bottom and top members are preferably parallel. Outwardly facing surfaces of the upstanding lateral members are preferably parallel to facingly contact preferably upstanding lateral members of adjacent units. Positioning the fabric in the curved shape shown permits a greater area of fabric per unit area occupied by the unite than the purely "rectangularly configured" unit illustrated in Figure 1.

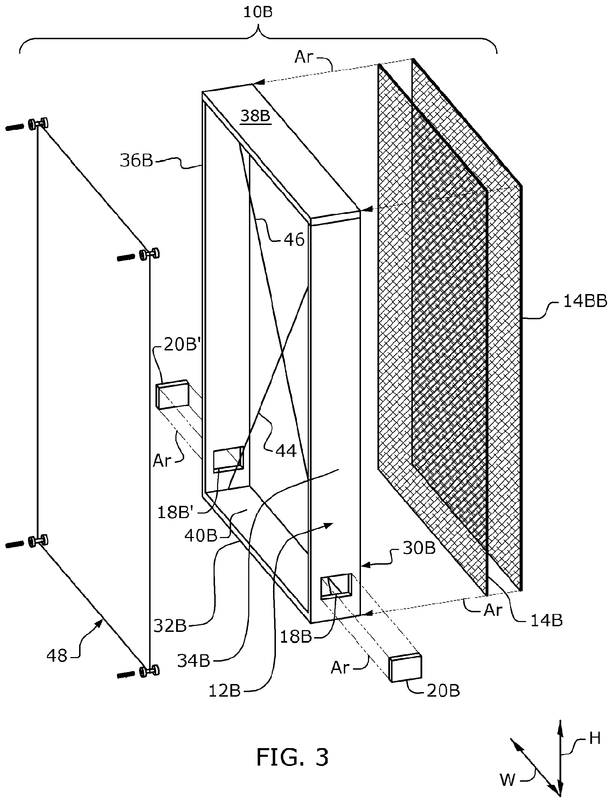

In another one of its aspects, as illustrated generally in Figure 3, the invention provides a preferably modular unit for improving indoor air quality where the unit includes a frame having an open center and defining the outer periphery of the unit, with the frame including a pair of parallel, spaced apart, lateral members connected by tensioning cables running from upper extremities of respective lateral members to lower extremities of respective opposed lateral members, and at least one aperture in at least one of the lateral members preferably opening in a direction perpendicular to the open center. The unit further preferably includes air permeable, preferably naturally occurring botanically based antimicrobial morbidity-inducing fabric secured about the frame periphery on a first side of the frame and covering the open center. The frame periphery on the remaining side of the frame is preferably planar and adapted to fit against a vertical wall. The unit includes a fan mounted in the aperture for blowing air from outside the frame into the frame interior for subsequent air passage from the frame interior outwardly through the fabric. The tensioning cables are adapted preferably to connect to one or more cable gripping devices preferably mounted in or on the vertical wall so that upon connection to the cable gripping devices, the tensioning cables preferably pull the planar remaining side of the frame against the vertical wall thereby closing the unit interior so that air may enter the unit only by action of the fan.

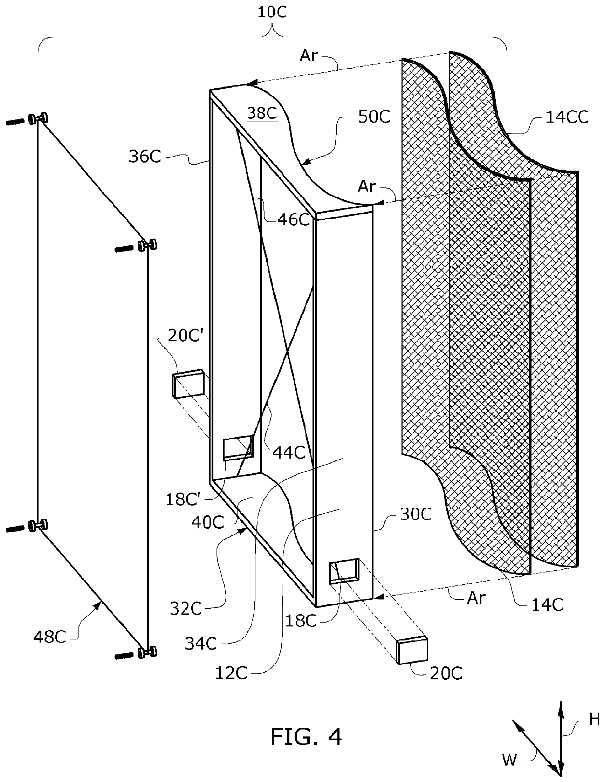

In still another one of its aspects, as illustrated generally in Figure 4, this invention provides a preferably modular unit for improving indoor air quality where the unit includes a frame surrounding an open interior and defining the outer periphery of the unit. Air permeable, preferably naturally occurring botanically based antimicrobial, morbidity-inducing fabric is secured about the frame periphery on a first side of the frame and covers the open interior on the first side of the frame. At least one aperture is formed in the frame. A fan is preferably mounted in the aperture and serves to blow air from outside the frame into the frame interior for subsequent air passage from the frame interior outwardly through the fabric with an optional second aperture, if provided, being a bleed air escape.

The unit is preferably mountable against a vertical wall by an upper portion of the frame preferably hanging on at least one member protruding from the wall with a second side of the frame being substantially flush with the wall so that air blown into the open interior by the fan escapes from the frame interior by passing through the fabric. The air permeable, preferably naturally occurring botanically based fabric preferably comprises multiple fabric layers and is preferably secured to the frame by Velcro. The frame preferably has four members with two of those members being spaced apart upstanding lateral members and the remaining members being bottom and top members. The frame is preferably rectangular, with the bottom and top frame members having edges, preferably defining top and bottom portions of the first side of the frame periphery, that may be curved. When curved, the edges are preferably in the form of an "S-curve"; the curved edges are preferably tangent to the upstanding members at juncture therewith, and edges of the bottom and top members are preferably parallel, all as shown generally in Figure 4. The curved edges allowed greater area of fabric than a unit with straight edges, as described above for the unit of Figure 2 vis-à-vis the unit of Figure 1.

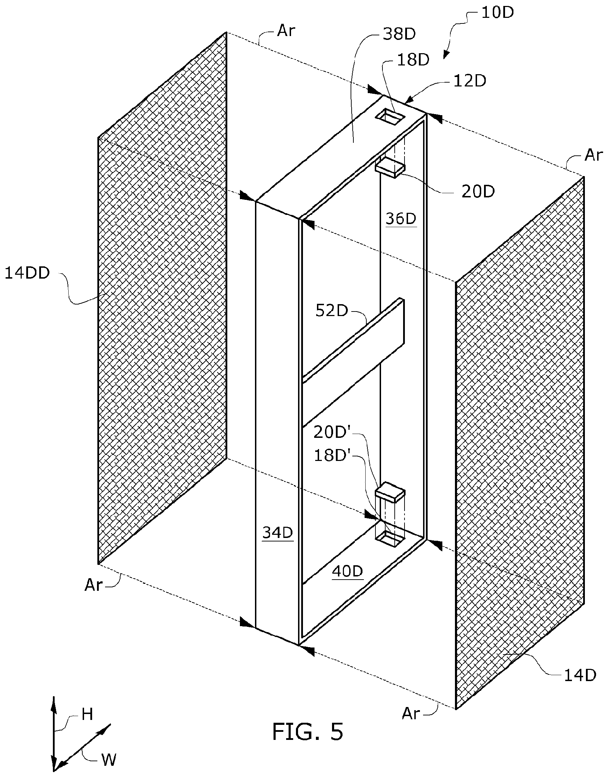

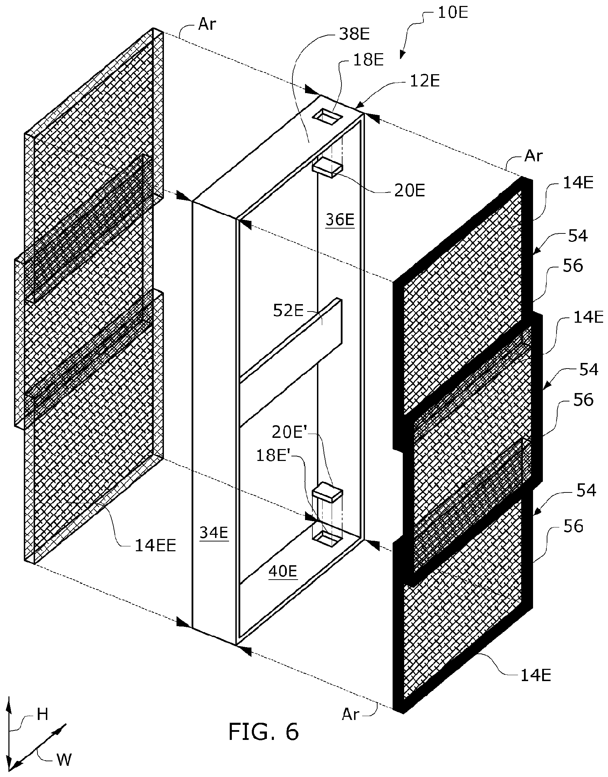

In still yet another aspect of the invention, as illustrated generally in Figure 5, there is provided a preferably modular unit for improving indoor air quality where the unit includes a frame surrounding an open interior and defining the outer periphery of the unit with air permeable, preferably naturally occurring botanically based antimicrobial, morbidity-inducing fabric secured about the frame periphery on first and second sides of the frame and covering the open interior on the first and second sides of the frame. A pair of apertures is preferably formed in the frame, one aperture in the upper extremity of the frame and the other aperture in the lower extremity of the frame. At least one fan is mounted on the frame and further is preferably aligned with one of the apertures, for drawing air from outside the frame into the frame interior through at least one of the aligned apertures for subsequent air passage from the frame interior outwardly through one of the two sections of fabric. The unit is preferably hung from the ceiling or other structure located well above the floor of a room; the unit may also sit on the floor. Air drawn into the open interior of the frame by the fan escapes from the frame interior by passing through one of the two sections of the fabric. The sections of air permeable fabric may comprise multiple fabric layers, preferably secured to the frame by Velcro as illustrated generally in Figure 6. Alternatively, the rear or second side of the frame may be closed, a per the unit illustrated in Figure 1. In either case, the frame preferably has four members with two of those being upstanding spaced-apart lateral members and the remaining members being bottom and top members. The frame is further preferably rectangular. The top and bottom members of the frame are preferably parallel. The unit may further include a brace extending between the upstanding spaced-apart lateral members proximate the vertical midpoints thereof. The outwardly facing lateral surfaces of the frame are preferably planar to facingly contact an outwardly facing lateral surface of the frame of an adjacent unit, preferably along the entire vertical length of the respective lateral surfaces.

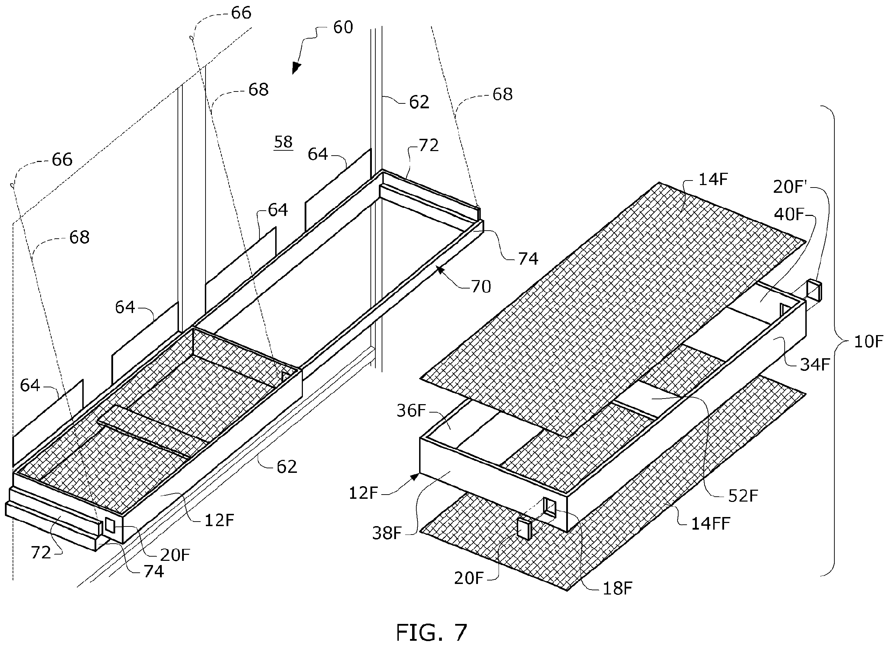

In still another one of its aspects, as illustrated generally in Figure 7, this invention provides a modular unit for improving indoor air quality where the unit includes a horizontal frame surrounding an open interior and defining a portion of the outer periphery of a filter portion of the unit. Air permeable, preferably naturally occurring botanically based antimicrobial, morbidity-inducing fabric is secured around the frame periphery on a downwardly facing side of the frame and covers the open interior of the downwardly facing side of the frame. Porous light-reflective fabric is preferably secured about the frame periphery on the upwardly facing side and covers the frame periphery on the upwardly facing side of the frame. At least one fan is mounted in the frame for blowing air from outside the frame into the frame interior for subsequent passage of air blown into the frame interior outwardly through the air permeable preferably naturally occurring botanically based fabric. An exterior portion of the frame outer periphery is preferably planar for facingly contacting a portion of a window. The unit further preferably includes at least one cable-like member extending upwardly from the frame for upwardly supporting the frame about a pivotal hook-and-eye connection at a position remote from the planar portion of the frame, preferably to permit weight of the frame to urge the frame against the window and maintain the frame in a horizontal plane.

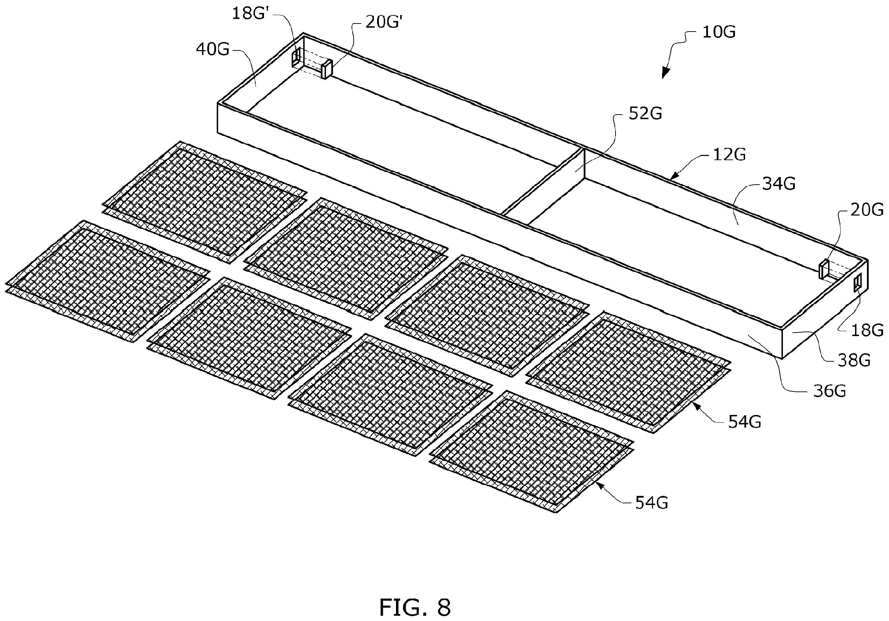

The unit further optionally includes at least one solar cell positionable to receive solar energy through the window, connected to and powering the fan. The air permeable fabric is preferably secured to the frame by Velcro; the frame preferably has four members; the frame is preferably rectangular; the vertically supporting member is preferably a cable and the downwardly facing surfaces of portions of the frame that are parallel to the window are preferably parallel with one another. Optimally, multiple layers of the antimicrobial fabric, constructed to be easily removable and in the form of replaceable modular assemblies, as illustrated generally in Figure 8, may also be used.

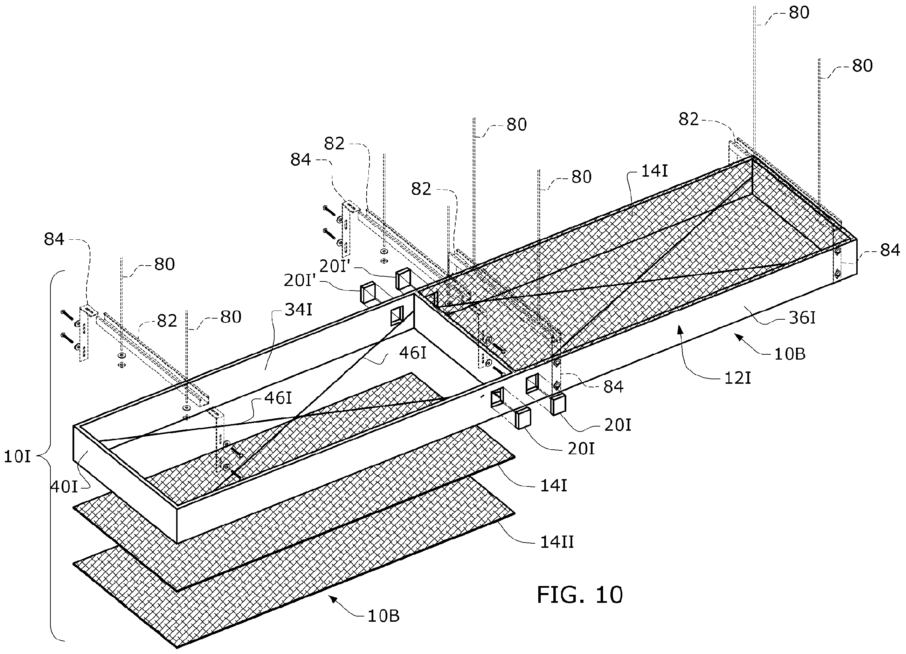

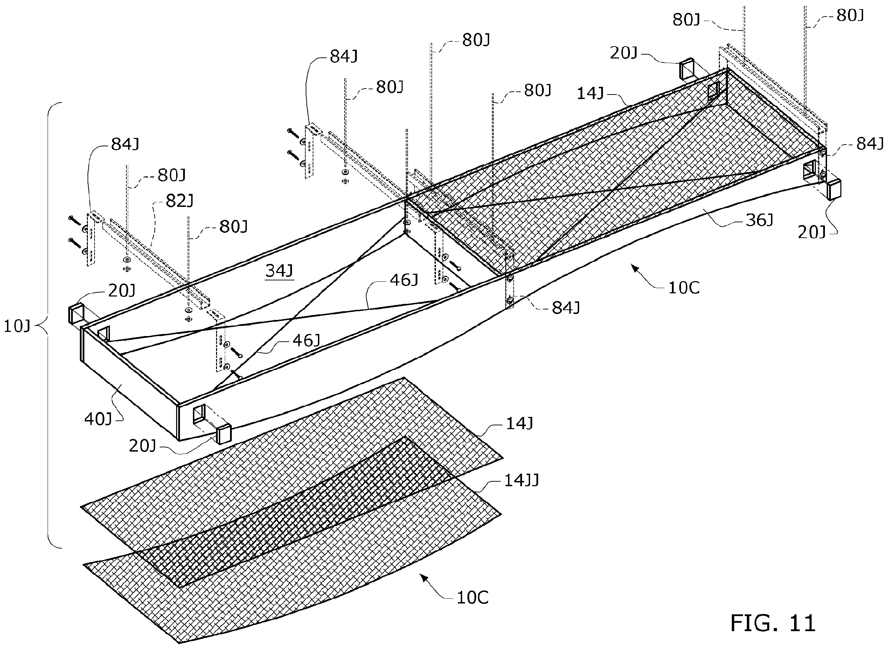

In still another one of its aspects, as illustrated generally in Figure 10, this invention provides a preferably modular unit for improving indoor air quality where the unit includes a frame surrounding an open interior and defining the outer periphery of the unit, air permeable preferably naturally occurring botanically based antimicrobial, morbidity-inducing fabric secured about the frame periphery on a lower side of the frame and covering the open interior on the lower side of the frame, and air porous preferably reflective preferably non-woven fabric secured about the frame periphery on the remaining upper side of the frame and covering the frame periphery on that remaining upper side. The unit has at least one aperture formed in the frame with the aperture adapted to house a fan therein. A fan is preferably mounted in the aperture for blowing air into the frame interior for subsequent passage of that air outwardly through the air permeable fabric. The unit further includes a pair of struts extending vertically from the frame for supporting the unit in mid-air within a room in a building. The fabric is preferably secured to the frame by Velcro; the frame is preferably rectangular and has four members, two being longer than the others; as illustrated in Figure 10, the unit preferably includes bracing cables within the frame interior extending between and affixed to respective ones of the longer members of the frame. In the unit, two parallel sides of the frame each have bottom edges that are parallel one with another. The lower edges of the frame may be gently curved, as shown in Figure 11, to enhance the aesthetics of the unit and to provide a larger area of the air permeable preferably naturally occurring botanically based antimicrobial, morbidity-inducing fabric for a given area occupied by the unit.

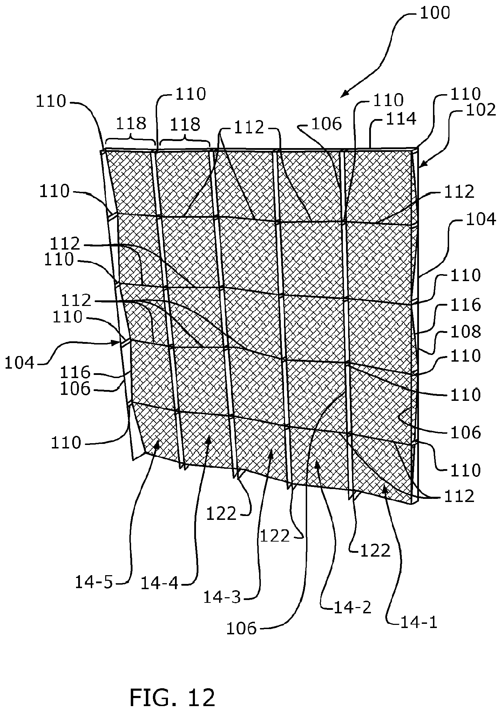

In still another one of its many aspects, as illustrated generally in Figure 12, the invention provides passive apparatus for improving indoor air quality, where the apparatus includes framework comprising a plurality of vertically upstanding members positioned at the corners of imaginary contiguous rectangles, one continuous edge of the contiguous rectangles being considered the rectangle fronts. Each of two pairs of the vertically extending members are associated with at least one of the rectangles. For each rectangle, one pair of members has a first one of its members at the right front of the rectangle and the second one of its members at the right rear of the rectangle, and a second pair of members has a first one of its members at the left front of the rectangle and a second one of its members at the left rear of the rectangle.

First and second horizontally oriented support members are preferably associated with respective ones of the rectangles and are preferably positionable on a floor or other surface to provide vertical support for the apparatus. These support members respectively preferably extend transversely between lower extremities of the first and second members of the respective pairs of upstanding members associated with the respective rectangle.

A plurality of vertically spaced apart pairs of parallel bracing members associated with respective ones of the rectangles connect respective ones of the upstanding first and second members of the respective pairs of upstanding member and lie along respective sides of the rectangle extending transversely away from the front. Uppermost lateral members associated with respective ones of the rectangles extend between and preferably slidably engage uppermost correspondingly positioned pairs of the horizontally extending parallel bracing members associated with respective rectangle.

Pluralities of lower lateral members below the uppermost lateral members associated with respective ones of the rectangles extend between and preferably slideably engage vertically corresponding positioned pairs of the preferably horizontally extending preferably parallel bracing members associated with the respective rectangle.

Air permeable, preferably naturally occurring botanically based antimicrobial, morbidity-inducing fabric is connected to at least one of the uppermost lateral members and extends downwardly always along and preferably between at least the vertically adjacent pairs of lower lateral members associated with respective rectangle(s). The lateral members are preferably movably positionable along the parallel bracing members, between front and rear, to cause fabric connected thereto and extending therefrom to conform to selected contours, with at least a portion of such contour preferably approximating the shape of the upper surface of an airfoil, so that the fabric assumes such contour in response to air blowing against the fabric. Optionally, a fan may be provided, desirably to blow air upwardly along the fabric.

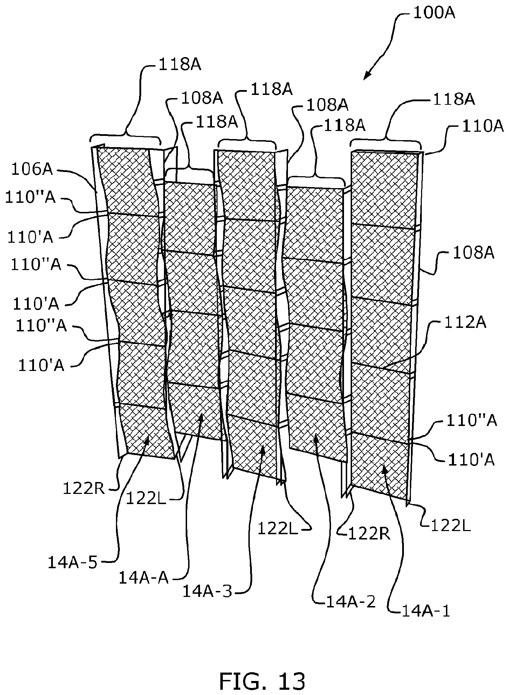

In still yet another one of its many aspects, as illustrated generally in Figure 13, the invention provides passive apparatus for improving indoor air quality where the apparatus includes a frame comprising vertically upstanding members positioned at the corners of an imaginary rectangle, one edge of the rectangle being considered the front of the rectangle. One pair of members has a first member of the pair at the right front of an associated rectangle and a second member of the pair at the right rear of the associated rectangle, with a second pair of members having a first member of the pair at the left front of the associated rectangle and a second member of the pair at the left rear of the associated rectangle. First and second horizontally oriented support members are preferably positionable on a floor or other surface to provide vertical support for the apparatus, and preferably respectively extend transversely between the lower extremities of the first and second members of the respective pairs of upstanding members. A plurality of vertically spaced apart pairs of parallel bracing members preferably connect respective ones of the upstanding first and second members of the respective pairs of upstanding members along respective sides of the rectangle, extending transversely away from the front. An uppermost lateral member extends between and preferably slideably engages uppermost correspondingly positioned pairs of the horizontally extending parallel bracing members. A plurality of lower lateral members below the uppermost lateral member, extend between and preferably slideably engage vertically correspondingly positioned pairs of the horizontally extending parallel bracing members. Air permeable, preferably naturally occurring botanically based antimicrobial, morbidity-inducing fabric is connected to the uppermost one of the lateral members and extends downwardly along and preferably between vertically adjacent pairs of lower lateral members. The lower lateral members are preferably movably positionable along the parallel bracing members, between front and rear, to cause the fabric portions connected thereto and extending therebetween to conform to selected contours. The lateral members may be positioned so that a portion of the resulting contour, or all of the resulting contour, approximates the upper surface of an airfoil, with the fabric conforming thereagainst in response to air blowing against the fabric. Similarly to the embodiment illustrated in Figure 12, optimally a fan may be provided to blow air along or against the fabric.

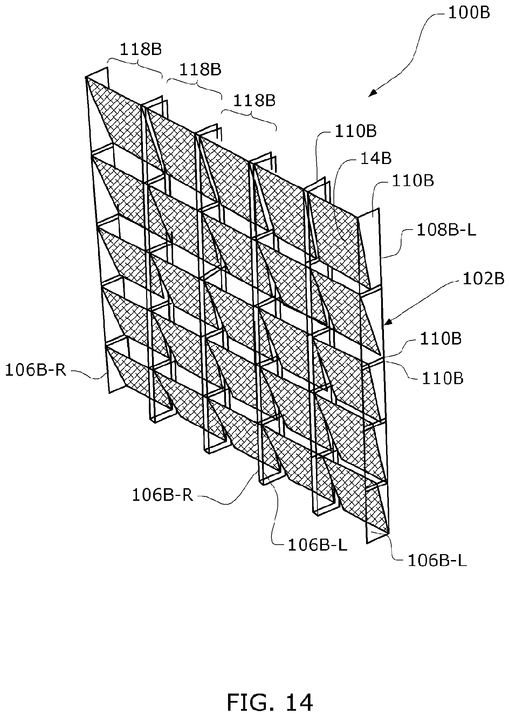

In still yet another one of its many aspects, as illustrated in generally in Figure 14, the invention provides passive apparatus for improving indoor air quality where the apparatus includes a frame comprising preferably vertically upstanding members preferably positioned at the corners of an imaginary rectangle, one edge of the rectangle being considered the front. One pair of members has a first member of the pair at the right front of the rectangle and the second member of the pair at the right rear of the rectangle, and a second pair of members has a first member of the pair at the left front of the rectangle and a second member of the pair at the left rear of the rectangle. Preferably, first and second horizontally oriented support members are preferably positionable on a floor or other surface to provide vertical support for the apparatus and respectively extend transversely between lower extremities of the first and second members of the respective pairs of upstanding members. A plurality of vertically spaced apart pairs of parallel bracing members preferably connect respective ones of the upstanding first and second members of the respective pairs of upstanding members along respective sides of the rectangle. Optionally, lateral members extend between and slideably engage vertically correspondingly positioned pairs of the horizontally extending parallel bracing members. Air permeable, preferably naturally occurring botanically based antimicrobial, morbidity-inducing fabric is connected to and extends between vertically adjacent pairs of bracing members. The optional lateral members are movably positionable along the parallel bracing members, between front and rear, to cause fabric portions to conform to selected contours, a portion of or all of which may approximate the upper surface of an airfoil, in response to air blowing against the fabric. Similarly to the embodiments illustrated in Figures 12 and 13, optionally a fan may be provided to blow air along or against the fabric.

### Brief Description of the Drawings

Figure 1 is an exploded isometric drawing of a first preferred embodiment of a modular unit for improving indoor air quality in accordance with aspects of the invention.
Figure 2 is an exploded isometric drawing of a second preferred embodiment of a modular unit for improving air quality in accordance with aspects of the invention.
Figure 3 is an exploded isometric drawing of a third preferred embodiment of a modular unit for improving indoor air quality in accordance with aspects of the invention.
Figure 4 is an exploded isometric drawing of a fourth preferred embodiment of a modular unit for improving indoor air quality in accordance with aspects of the invention.
Figures 5 and 6 are exploded isometric drawings of preferred fifth and sixth preferred embodiments of modular units for improving indoor air quality in accordance with aspects of the invention.
Figure 7 is an exploded isometric drawing of a seventh preferred embodiment of a modular unit for improving indoor air quality in form of a breathing light shelf, in accordance aspects of the invention.
Figure 8 is an exploded isometric drawing of an eighth preferred embodiment of a modular unit for improving indoor air quality in the form of a breathing light shelf, similar to that shown in figure 7, in accordance with aspects of the invention.
Figure 9 is an exploded isometric drawing of a ninth preferred embodiment of a modular unit for improving indoor air quality in the form of a breathing light shelf, similar to the embodiments illustrated in figures 7 and 8, in accordance with aspects of the invention.
Figure 10 is an exploded isometric drawing of a modular unit for improving indoor air quality in the form of a suspended breathing light shelf in accordance with aspects of the invention.
Figure 11 is an exploded isometric drawing of a modular unit for improving indoor air quality in the form of a suspended breathing light shelf, similar to that illustrated in figure 10, in accordance with aspects of the invention.
Figure 12 is an isometric drawing of a twelfth preferred embodiment of apparatus for preferably passively improving indoor air quality in the form of an upstanding modular vertically suspended fabric array, in accordance with aspects of the invention.
Figure 13 is an isometric drawing of a thirteenth preferred embodiment of apparatus for preferably passively improving indoor air quality in the form of an upstanding modular vertically suspended fabric array, similar to that illustrated in Figure 12, in accordance with aspects of the invention.
Figure 14 is an isometric drawing of a fourteenth preferred embodiment of apparatus for preferably passively improving indoor air quality in the form of an upstanding modular vertically suspended fabric array, similar to that illustrated in Figures 12 and 13.
Figure 15 is a broken isometric drawing of one of the five vertically extending segments of the apparatus for preferably passively improving indoor air quality illustrated in Figure 12.
Figure 16 is a broken isometric drawing of one of the five vertically extending segments of the apparatus for preferably passively improving indoor air quality illustrated in Figure 14.

### Detailed Description of the Preferred Embodiments of the Invention and the Best Mode Known at the Present Time for the Practice of the Same

Referring generally to the drawings and specifically to Figure 1, apparatus for treating and alleviating SBS to improve indoor air quality is depicted in the form of a modular unit designated generally 10 that includes a frame designated generally 12 surrounding an open interior and defining an outer periphery of unit 10. An air permeable, antimicrobial, morbidity-inducing fabric 14, preferably comprising a naturally occurring antimicrobial botanical compound, is secured about the periphery of frame 12, on a first side 30 of frame 12, with fabric 14 covering the open interior of frame 12 on the first side 30 of frame 12.

An air impermeable member designated generally 16 is secured about the periphery of frame 12 on a remaining or second side 32 of frame 12. Air impermeable member 16 covers the periphery of frame 12 on second side 32 of frame 12 completely about the frame periphery.

At least one aperture 18 is formed in frame 12. Aperture 18 is adapted to house a fan 20 therein. Fan 20 is depicted schematically in Figure 1 and is preferably battery-powered and self-controlling. A second aperture 18' may also be provided, as illustrated, to house a second optional fan 20' or may be at least partially open and used for air bleed.

Fan 20, being housed in aperture 18, serves to blow air from outside of frame 12 into the interior of frame 12 for subsequent passage of substantially all air blown into the frame interior, outwardly through fabric 14.

In viewing Figure 1, arrows identified by letters "Ar" indicate the manner of assembly of unit 10, which is shown in Figure 1 in a partially exploded isometric view.

Still referring to Figure 1, frame 12 has four members, two which are first and second upstanding lateral members 34 and 36, which are spaced apart as illustrated in Figure 1; the remaining two members of frame 12 are a top member 38 and a bottom member 40.

Frame 12 further includes a bracing member 42 that is preferably upstanding and extends between and is affixed both to bottom member 40 and to top member 38 of frame 12. Bracing member 42 is preferably affixed to both top member 38 and bottom member 40 by screws, or by suitable adhesive, or by other mechanical fastening means. Similarly, frame 12 of unit 10 is preferably assembled from particle board or wood using adhesive or screws or other mechanical means to secure the parts of frame 12 together in the manner indicated by arrows Ar in Figure 1. The screws, adhesive or other mechanical means used in the assembly of frame 12 have not been illustrated in Figure 1, to enhance clarity of the drawing. As seen in Figure 1, frame 12 is preferably of generally rectangular configuration, with frame 12 preferably being higher than it is wide.

The air permeable, antimicrobial, morbidity-inducing fabric 14, preferably comprising a naturally occurring antimicrobial botanical compound, is preferably secured about the edges of frame 12 that face fabric 14 when fabric 14 and frame 12 are oriented in the position illustrated in Figure 1. Velcro is preferably used to secure fabric 14 to the surfaces of frame 12 that face fabric 14 when those parts are oriented as illustrated in Figure 1. The Velcro has not been illustrated to enhance clarity of the drawing. Use of Velcro facilitates replacement of fabric 14 on a periodic basis.

When unit 10 is assembled by putting the parts of frame 12 in place as indicated by arrows Ar, including by positioning motor 20 within aperture 18, and by attaching fabric 14 to the facing edges of frame 12 using the preferable Velcro, other than for the presence of upstanding bracing member 42, the interior of frame 12 is open.

Upstanding bracing member 42 is of width substantially less than the width of the top and bottom 38 and 40 of frame 12, where width is considered in the direction indicated by arrow W in Figure 1 and height is considered in the direction indicated by arrow H in Figure 1. With upstanding bracing member 42 being of width substantially less than top and bottom 38 and 40 of frame 12, when frame 12 is assembled, the interior of frame 12 is essentially open, thereby providing a plenum that is at least partially bounded by fabric 14. When fan 20 operates, fan 20 introduces air into the plenum defined by the interior of unit 10 and forces air gently outwardly through fabric 14. Fabric 14 is air permeable and has antimicrobial, morbidity-inducing characteristics due to having been treated with and preferably even impregnated with a naturally occurring antimicrobial botanical material, preferably clove powder or eugenol. Hence, when room air is forced gently into the open interior of unit 10, which defines a plenum, and then outwardly through fabric 14, airborne bacteria and other contaminants are trapped and killed by fabric 14.

As further apparent from Figure 1, frame 12 has a generally rectangular configuration such that first side 30 and second side 32 are parallel one with another, and such that top 38 and bottom 40 are parallel one with another. Additionally, the edges, which are unnumbered in the drawings, of first and second sides 30, 32 and top and bottom 38, 40, are all coplanar, thereby presenting a flat, rectangular, frame-like surface for preferable adhesive affixment of the Velcro male or female portion that mates with the counterpart Velcro portion affixed to fabric 14. Fabric 14 is preferably of rectangular shape and dimensioned to fit congruently with the facing edges of first and second sides 30, 32 and top and bottom 38, 40, defining the rectangular shape of frame 12, so that there is no substantial overlap of fabric 14 respecting frame 12, and so that there is no opening between an edge of fabric 14 and a portion of frame 12 through which air could escape without passing through fabric 14.

From the foregoing description of the structure illustrated in Figure 1, together with the image provided by Figure 1, it is apparent that unit 10 may be used to combat SBS and includes a plenum defined by the interior of frame 12, which is at least partially bounded by fabric 14 where fabric 14 has at least one preferably naturally occurring botanical substance exhibiting antimicrobial, microorganism morbidity-inducing properties, and where unit 10 further includes a fan for introducing air into the plenum for passage of the air outwardly through the fabric, thereby to trap and to kill airborne bacteria and other contaminants contributing to SBS. It is further apparent that operation of unit 10 inherently practices a method for combating SBS by providing a plenum, namely the interior of frame 12, which is at least partially bounded by fabric, namely the air permeable, antimicrobial, morbidity-inducing fabric 14, preferably comprising a naturally occurring antimicrobial botanical compound, where the method further includes introducing air into the plenum for passage outwardly through the fabric. It is still further apparent that operation of unit 10 inherently practices a method for combating SBS by forcing air from a room contaminated by SBS through a fabric where the fabric has at least one preferably naturally occurring botanical substance exhibiting microorganism morbidity-inducing properties.

Still referring generally to the drawings and specifically to Figure 2, apparatus for treating and eliminating SBS and improving indoor air quality is depicted in the form of a modular unit designated generally 10A. Unit 10A is similar to unit 10 illustrated in Figure 1 with the letter "A" used in Figure 2 to identify the apparatus illustrated in Figure 2. Components illustrated in Figure 2 having the same function or effectively identical to components in Figure 1 have the same numbers in Figure 2 as in Figure 1.

The apparatus illustrated in Figure 2 differs from the apparatus illustrated in Figure 1 in that the top 38A of frame 12A and the bottom 40A of frame 12A, have edges that are not straight as illustrated in Figure 1 but are curved in somewhat the shape of an "S" curve, as the outwardly facing edge of top 38A is clearly visible in Figure 2. This forwardly or outwardly facing edge of top 38A is designated 50A in Figure 2. Providing forwardly facing edge 50A in a curved, desirable S-shaped configuration permits use of air permeable, antimicrobial, morbidity-inducing fabric 14A, preferably comprising a naturally occurring antimicrobial botanical compound, having greater surface area in the embodiment illustrated in Figure 2 than in the embodiment illustrated in Figure 1, with the footprint of unit 10A, namely the floor space occupied by unit 10A, remaining the same as the footprint of unit 10 illustrated in Figure 1. As a result, unit 10A in Figure 2 may have greater air treatment capacity for a given amount of floor space occupied by unit 10A than the air treatment capacity of unit 10, as illustrated in Figures 2 and 1, respectively. Additionally, the forwardly facing edge 50A with the curved, S-shaped configuration provides even greater aesthetics for unit 10A illustrated in Figure 2 relative to unit 10 illustrated in Figure 1.

Still referring generally to the drawings and specifically to Figure 3, another embodiment of apparatus for treating and alleviating SBS thereby improving indoor air quality is depicted in the form of a modular unit designated generally 10B that includes a frame designated generally 12B surrounding an open interior and defining an outer periphery of unit 10B. In Figure 3, the same lettering and numbering convention as used in Figures 1 and 2 has been employed. Specifically, components having the same or substantially the same function and generally corresponding substantially to components illustrated in Figures 1 and 2 have the same number; the letter "B", or in some cases the letters "BB", is used to denote Figure 3 and the embodiment therein and to distinguish the same from the embodiments illustrated in Figures 1 and 2. In Figure 3, two layers 14B and 14BB of air permeable, antimicrobial, morbidity-inducing fabric, preferably comprising a naturally occurring antimicrobial botanical compound are secured about the periphery of frame 12B on a first side 30B of frame 12B with fabric 14B facingly contacting the open interior of frame 12B on first side 30B of frame 12B, and with fabric 14BB facingly contacting fabric 14B and lying congruently thereover.

Similarly to the apparatus illustrated in Figures 1 and 2, at least one aperture 18B is formed in frame 12B. Aperture 18B is adapted to house a fan 20B therein. Fan 20B is depicted schematically in Figure 3. A second aperture 18B' may also be provided as illustrated to house a second optional fan 20B' or may be used for air bleed.

Fan 20B, being housed in aperture 18B, serves to blow air from outside of frame 12B into the interior of frame 12B for subsequent passage of substantially all air that is blown into the frame interior, outwardly through fabric 14B and 14BB.

As with Figures 1 and 2, the arrows identified in Figure 3 by letters "Ar" indicate the manner of assembly of unit 10B, which is shown in Figure 3 in a partially exploded isometric view.

The remaining or second side 32B of frame 12B may be open as illustrated, or may be covered with one or more layers of air permeable, antimicrobial, morbidity-inducing fabric.

Still referring to Figure 3, frame 12B has four members, two of which are first and second upstanding lateral members 34B and 36B, which are spaced apart as illustrated in Figure 3; the remaining two members of frame 12B are top member 38B and bottom member 40B.

Frame 12B further preferably includes first and second diagonal bracing cables 44 and 46, each of which extend from a lower interior corner of frame 12B, defined by juncture of bottom 40B and upstanding side member 34B or 36B, to a diagonally opposite upper corner, defined by juncture of top 38B with either upstanding side member 36B or upstanding side member 34B. Diagonal bracing cables 44 and 46 are secured in place, desirably by connecting with eyes driven into the wood or particle board construction, at a location close to if not exactly at the line of juncture between the top and bottom members 22B, 24B and the respective side members 34B, 36B. The eyes and the particular securement of diagonal bracing cables 44 and 46 to frame 12B have not been illustrated in Figure 3 to enhance drawing clarity.

Unlike the unit illustrated in Figure 1, the remaining or second side 32B of frame 12B in the unit illustrated in Figure 3 has been illustrated open. Unit 10B is preferably equipped with a hanging cable 48 connected to second side 32B of frame 12B by suitable screw and collar assemblies, which have not been detailed or numbered in Figure 3 to enhance drawing clarity. As shown in Figure 3, screws are driven into the second side 32B of frame 12B at the four corners of second side 32B and collars are then secured in place by screws and permit a small degree of movement of hanging cable 48. Presence of hanging cable 48 facilitates hanging unit 10B on and against a wall, with the wall thereby effectively closing second side 32B of frame 12B in much the same manner as air permeable member 16 covers the periphery of frame 12 on the second side 32 of frame 12 as illustrated in Figure 1.

Hanging cable 48 and the unnumbered screws and collars that connect hanging cable 48 to the remainder of the structure may also and optionally be positioned to maintain the frame 12B slightly away from the wall on which unit 10B is mounted. This is desirable if the remaining or second side 32B of frame 12B is covered with one or more layers of air permeable, antimicrobial, morbidity-inducing fabric, preferably comprising a naturally occurring antimicrobial botanical compound. Unit 10B, using hanging cable 48, can be mounted against any reasonably imperforate wall surface; provision of hanging cable 48 permits unit 10B to be mounted essentially flush against the surface of the wall on which unit 10B is mounted. Molly bolts, hooks or the like, driven into a wall may be used to hang unit 10B on the wall.

While unit 10B has been illustrated with two thicknesses of air permeable, antimicrobial, morbidity-inducing fabric 14B and 14BB, a single fabric thickness may be used, depending on the amount of air moved by fan 20B as selected in specifying fan 20B. While one or more layers of air permeable, antimicrobial, morbidity-inducing fabric, preferably comprising a naturally occurring antimicrobial botanical compound, 14B, 14BB may be used on the front and rear surfaces of frame 12B, an aesthetically pleasing, air permeable fabric lacking antimicrobial and morbidity-inducing properties may be used as the outermost fabric 14BB to enhance the aesthetics of unit 10B.

Similarly to the unit illustrated in Figure 1, frame 12B of unit 10B is preferably assembled from particle board or wood using adhesive, screws or other mechanical means to secure the parts of frame 12B together in the manner indicated by arrows Ar in Figure 3. As in Figure 1, the screws, adhesive or other mechanical means used in the assembly of frame 12B have not been illustrated in Figure 3 to enhance clarity of the drawing. Similarity to the apparatus illustrated in Figures 1 and 12, frame 12B is preferably of generally rectangular configuration with frame 12 preferably being higher than it is wide.

The air permeable, antimicrobial, morbidity-inducing fabric 14B is preferably secured about the edges of frame 12B that face fabric 14B when fabric 14B and frame 12B are oriented in the position illustrated in Figure 3. As with the apparatus illustrated in Figures 1 and 2, Velcro is preferably used to secure fabric 14B to the surfaces of frame 12B that face fabric 14B when those parts are oriented as illustrated in Figure 3. Similarly, Velcro is preferably used to secure fabric 14BB to the surface of fabric 14B when those fabric layers are oriented as illustrated in Figure 3. The Velcro has not been illustrated in order to enhance the drawing. Use of Velcro facilitates replacement of the fabrics on a periodic basis.

Similarly to the apparatus illustrated in Figures 1 and 2, when unit 10B is assembled by putting the parts of frame 12B in place as indicated by arrows Ar, by positioning motor 20B with an aperture 18B, and by attaching fabric 14B and 14BB to the facing edges of frame 12B using the preferable Velcro, and unit 10B is either mounted flushly against a wall or has fabric 14BBB covering the rear or second side of unit 10B, the interior of frame 12B is open other than for the presence of diagonal bracing cables 44, 46. The open construction provides a plenum that is at least partially bounded by fabric 14B. When fan 20 operates, fan 20 introduces air into the plenum defined by the interior of unit 10B and forces air gently outwardly through fabric 14B and fabric 14BB. Fabrics 14B and 14BB are both air permeable and preferably each has antimicrobial, morbidity-inducing characteristics due to having been treated with, and even impregnated with, a naturally occurring botanical, antimicrobial, morbidity-inducing material, preferably clove powder or eugenol. Hence, when room air is forced gently into the open interior of unit 10B, which defines a plenum, and then outwardly through fabric 14B, 14BB, airborne bacteria and other contaminants are trapped and killed by fabric 14B and 14BB.

As also apparent from Figure 3, frame 12B has a generally rectangular configuration such that first side 30B and second side 32B are parallel one with another and such that top 38B and bottom 40B are parallel one with another. Additionally, the edges, which are unnumbered in the drawings, of the first and second sides 30B, 32B and top and bottom 38B, 40B are all coplanar, thereby presenting a flat, rectangular, frame-like surface for preferable adhesive securement of the Velcro male or female portion that mates with the counterpart Velcro portion affixed to fabric 14B. Fabric 14B and fabric 14BB are both preferably rectangularly shaped and dimensional to fit congruently with the facing edges of first and second sides 30B, 32B and the facing edges of top and bottom 38B, 40B defining the rectangular shape of frame 12B so there is no substantial overlap of fabric 14B, 14BB, respecting frame 12, and so there is no opening between an edge of fabric 14B and a portion of frame 12B through which air could escape without passing through fabric 14B.

Similarly to the apparatus illustrated in Figures 1 and 2, from the foregoing description in combination with the image provided by Figure 3, it is apparent that unit 10B may be used to combat SBS and includes a plenum defined by the interior of frame 12B when frame 12B is closely spaced and perhaps even contacting the wall on which frame 12B is mounted, with the plenum at least being partially bounded by fabric 14B, where fabric 14B has at least one preferably naturally occurring botanical substance exhibiting antimicrobial, microorganism morbidity-inducing properties, and where unit 10B further includes a fan for introducing air into the plenum for passage of the air outwardly through the fabric, thereby to trap and kill airborne bacteria and other contaminants contributing to SBS. It is further apparent that operation of unit 10B inherently practices a method for combating SBS by providing a plenum, namely the interior of frame 12B, which is at least partially bounded by fabric, namely the air permeable, antimicrobial, morbidity-inducing fabric 14B, where the method further includes introducing air into the plenum for passage outwardly through the fabric.

Still referring generally to the drawings and specifically to Figure 4, yet another embodiment of apparatus for treating and alleviating SBS thereby improving indoor air quality is depicted in the form of a modular unit designated generally 10C that includes a frame designated generally 12C surrounding an open interior and defining an outer periphery of unit 10C. In Figure 4, the same lettering and numbering convention has been used for the apparatus therein as used above for the apparatus illustrated in Figures 1, 2 and 3, in that elements or components of apparatus 10C that are identical to or functionally corresponding with elements or components in Figures 1, 2 and 3 have the same number, with letter "C" identifying and distinguishing the apparatus illustrated in Figure 4 from the apparatus illustrated in Figures 1, 2 and 3.

In apparatus 10C illustrated in Figure 4, much like apparatus 10A illustrated in Figure 2, the forwardly facing edges of top 38C and bottom 40C of frame 12C are curved, preferably in an S-shape configuration, as illustrated by the forwardly facing edge 50C of top 38C in Figure 4. This, similarly to the curved forwardly facing edge configuration in Figure 2, facilitates use of a larger surface area of fabric 14C, 14CC, in a given floor area, where the footprint of unit 10C is the same as that of unit 10B. In all other respects, the descriptive material set forth above respecting Figure 3 is incorporated herein by reference with respect to Figure 4.

Still referring generally to the drawings and specifically to Figure 5, yet another embodiment of apparatus for treating and alleviating SBS and thereby improving indoor air quality is depicted in the form of a modular unit designated generally 10D that includes a frame designated generally 12D surrounding an open interior and defining an outer periphery of unit 10D. The same numbering convention has been used in Figure 5 as used in Figures 1 through 4 in that elements of apparatus 10D that are identical to or functionally correspond with elements in Figures 1 through 4 have the same numbers and the letter "D" is used to identify and distinguish parts and components of unit 10D as illustrated in Figure 5 from parts and components illustrated in Figures 1 through 4.

Unit 10D illustrated in Figure 5 differs from the apparatus illustrated in Figures 1 through 4 in that the apparatus illustrated in Figure 5 is not intended to rest on the floor, but rather is intended to be hung in a freestanding manner within a room requiring remediation and cure of SBS symptoms. As illustrated in Figure 5, bottom member 24D of frame 12D is equipped with an aperture 18D' into which a fan 20D' may be positioned. Similarly, top member 22D of frame 12D includes an aperture 18D into which a fan 20D may be positioned. Additionally, unit 22D preferably includes two air permeable, antimicrobial, morbidity-inducing fabrics, preferably comprising a naturally occurring antimicrobial botanical compound, 14D and 14DD, with one fabric fitting on each side of frame 12D. Arrows Ar identify the manner in which unit 10D is assembled with fabrics 14D, 14DD fitting against frame 12D. Not all of the arrows denoting such assembly have been labeled "Ar" in order to enhance drawing clarity. Frame 12D further includes horizontal interior bracing member 52D to provide rigidity to frame 12D when assembled. With the exception of horizontal interior bracing member 52D, the interior of frame 12D is open when unit 12D is assembled.

As respecting operation of unit 10D, the information and description as provided above as respecting units 10, 10A, 10B and 10C illustrated in Figures 1 through 4 is hereby incorporated by reference as being equally applicable to unit 10D illustrated in Figure 5.

Still referring generally to the drawings and specifically to Figure 6, yet another embodiment of apparatus for treating and alleviating SBS thereby improving indoor air quality is depicted in the form of a modular unit designated generally 10E that includes a frame designated generally 12E surrounding a generally open interior and defining an outer periphery of unit 10E. In Figure 6, the same numbering and lettering convention as used with respect to the apparatus illustrated in Figures 1 through 5 has been adopted; in Figure 6, the letter "E" identifies and distinguishes components of unit 10E illustrated therefrom from the corresponding parts and components of the apparatus illustrated in Figures 1 through 5. Referring still to Figure 6, frame 12E and the parts thereof, namely top member 22E, bottom member 24E, lateral members 26E, horizontal interior bracing member 52E, fans 20E and 20E' and apertures 18E and 18E' are preferably substantially identical to the correspondingly numbered components of unit 10D illustrated in Figure 5.

In Figure 6, the air permeable, antimicrobial, morbidity-inducing fabric is furnished in the form of modular fabric panels designated generally 54 in Figure 6, where each modular fabric panel includes a frame 56 that is generally of rectangular construction with an open center. Preferably two layers of air permeable, botanically based, antimicrobial, morbidity-inducing fabric 14E and 14EE are a part of each modular fabric panel 54 with a first layer of fabric 14E secured to one side of frame 56 and a second layer of fabric 14EE secured to a second side of frame 56, where the fabric in both instances is preferably secured to frame 56 using Velcro. In Figure 6, to enhance drawing clarity, the frames 56 of modular fabric panels 54 have been illustrated only for modular fabric panels 54 on the right side of the drawing. Similarly, fabric layer 14E has been designated only for those modular fabric panels on the right side of the drawing and fabric layer 14EE has been designated only for those modular fabric panels on the left side of the drawing.

Each modular fabric panel preferably includes two layers of fabric, one on either side of fabric panel frame 56. Modular fabric panels 54 may be dimensioned such that when mounted on frame 12E there is some overlap of the upper and lower panels by the middle panel as illustrated in Figure 6; unit 10E may also be constructed such that modular fabric panels 54 all collectively fit flushly one against another on one side of frame 12E to present a smooth, continuous surface of air permeable, antimicrobial, morbidity-inducing fabric, preferably comprising a naturally occurring antimicrobial botanical compound, for passage of treatment air therethrough.

As respecting the operation of unit 10E and the components thereof, the information and disclosure as set forth above with respect to the apparatus illustrated in Figures 1 through 5 is incorporated by reference.

Still referring generally to the drawings and specifically to Figure 7, yet another embodiment of apparatus for treating and alleviating SBS and improving indoor air quality is depicted in the form of a modular unit designated generally 10F that includes a frame designated generally 12F surrounding an open interior and defining an outer periphery of unit 10F. As with the apparatus illustrated in Figures 1 through 6, the same numbering and lettering convention has been used with the letter "F" being used to identify and distinguish components of the apparatus illustrated in Figure 7 from corresponding functionally and substantially equivalent components of the apparatus illustrated in Figures 1 through 6. As illustrated in the right-hand, exploded view portion of Figure 7, the apparatus for treating and alleviating SBS and improving indoor air quality appearing there and indicated as 10F is substantially identical to that illustrated in Figure 5 and designated 10D. In Figure 7, apparatus 10F has been illustrated in a horizontal disposition and, as shown in the left hand portion of Figure 7, is adapted to be used in such a horizontal orientation.

As further illustrated in the left-hand portion of Figure 7, unit 10F is preferably mounted in a horizontal disposition on a unit support frame designated generally 70 that is positioned within a structure designated generally 60 and preferably is in essentially facing contact with the interior surface of a window, or at least the frame of the window, designated generally 58. Unit support frame 70 is preferably maintained in place and vertically supported by cable 68 preferably connected to hooks 66 mounted in the interiorly facing surface of wall 62, above window 58.

Unit support frame 70 preferably includes an inner member designated generally 72 and an outer member designated generally 74 as shown in the left-hand portion of Figure 7. Outer member 74 is dimensioned to vertically support unit 10F by contact with a downwardly facing portion thereof, preferably the downwardly facing portion of frame 12F of unit 10F, as illustrated at the extreme left-hand side of Figure 7. Inner member 72 of unit support frame 70 is dimensioned to receive unit 10F in a facing, complemental manner with unnumbered vertically extending, horizontally facing surfaces of inner member 72 facingly contacting the interiorly positioned one of lateral members 26F and members 22F and 24F. The portion of inner member 72 extending essentially perpendicularly inwardly from window 58 is dimensioned to stop short of the position of fan 20F in aperture 18F, all as illustrated in the extreme left-hand portion of Figure 7.

Solar cells 64 are preferably positioned in facing contact with window 58 to receive sunlight and thereby generate electricity. Solar cells 64 are connected by wires, not shown in the drawings, to fans 20F so that fans 20F are driven by solar energy received through window 58, such that batteries may not be required for fans 20F.

In one preferable implementation of the invention as illustrated in Figure 7, fabric 14F on the upper side of unit 10F may be a non-woven fabric that is not only air permeable and antimicrobial with morbidity inducing properties, but is also reflective in a manner to reflect natural light coming in through window 58 throughout the room in which unit 10F is mounted. Distribution of natural light within a room having SBS symptoms serves to alleviate those symptoms and in combination with the air purification effectuated by unit 10F provides synergistic results as respecting elimination of SBS.

Still referring generally to the drawings and specifically to Figure 8, apparatus for treating and alleviating SBS and improving indoor air quality is depicted in the form of a modular unit designated generally 10G that includes a frame designated generally 12G surrounding an open interior and defining an outer periphery of unit 10G. Unit 10G is designed to fit on a unit support frame 70 illustrated in Figure 7. The frame 12G of unit 10G is essentially identical to frame 12F illustrated in Figure 7 except for the position of interior bracing member 52G which has been positioned to be in the same plane with and transverse to lateral members 26G of frame 12G.

As further illustrated in Figure 8, the air permeable antimicrobial, morbidity inducing fabric, preferably comprising a naturally occurring antimicrobial botanical compound, is provided in the form of modular fabric panels 54G, as shown in Figure 6 as panels 54. Frames of the modular panels 54G have not been illustrated in Figure 8 to enhance drawing clarity. The same numbering convention has been used with respect to unit 10F illustrated in Figure 8 as for the apparatus illustrated in Figures 1 through 7 and the disclosure as set forth above for the apparatus illustrated in Figures 1 through 7 and the operation thereof is hereby incorporated as applicable to apparatus 10G illustrated in Figure 8. Not all of modular fabric panels 54G have been so- designated in Figure 8 to enhance drawing clarity.

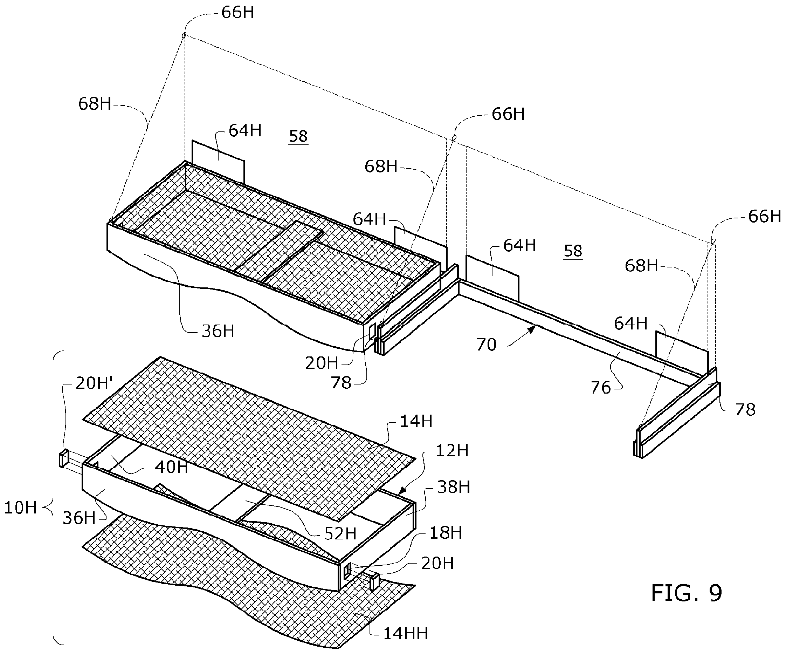

Still referring generally to the drawings and specifically to Figure 9, apparatus for treating and alleviating SBS and improving indoor air quality is depicted in the form of a modular unit designated generally 10H that is similar to that illustrated in Figure 7, with the exception that downwardly facing edges of lateral members 26 are formed in an S-curve in order to provide more surface area of air permeable, antimicrobial, morbidity-inducing fabric, preferably comprising a naturally occurring antimicrobial botanical compound, 14H, than the fabric surface area provided by the embodiment illustrated in Figures 7 for a given area occupied by unit 10H. In all other respects apparatus illustrated in Figure 9 as unit 10H is essentially identical to that illustrated in Figure 7 as apparatus 10F, and the disclosure as set forth above respecting the apparatus of Figure 7 is hereby substantially incorporated by reference with respect to the apparatus illustrated in Figure 9. One exception to the incorporation by reference is a minor change to the configuration of unit support frame 70 in that outer member 74 illustrated in Figure 7 does not extend across the front of unit support frame 70, as illustrated in Figure 9. Rather, unit support frame 70 as illustrated in Figure 9 includes a bottom support member 76 for supporting unit 10H by facing complemental contact with the lower surfaces of three sides of the frame 12H of unit 10H. Unit support frame 70 as illustrated in Figure 9 includes a pair of lateral guide members 78 which facingly contact members 22H, 24H, as illustrated in the upper portion of Figure 9, but are dimensioned so as to stop short of fan 20H, as illustrated in the upper portion of Figure 9.

Continuing to refer to the drawings, Figures 10 and 11 illustrate use of the apparatus for treating and alleviating SBS thereby improving indoor air quality, as depicted in Figures 3 and 4 and identified as units 10B and 10C, in a horizontal orientation with the units being suspended in mid-air from the ceiling or other structure in a room in order to alleviate SBS. In Figure 10, two units 10B are illustrated in side-by-side disposition, with one of the units, on the left side of Figure 10, being shown with an open center. In use, the upper surface of unit 10B illustrated to the left in Figure 10 would be covered, either with one or more layers of air permeable, antimicrobial, morbidity inducing fabric 14 or with a solid cover such as rear cover member 16 illustrated in Figure 1.

Figure 11 similarly shows two units 10C with the unit to the left-hand side of Figure 11 having an open top which would be covered in the same manner as that of Figure 10. In Figures 10 and 11, the composite units 10B, in the case of Figure 10, or two units 10C in the case of Figure 11, have been designated 10I and 10J respectively. In Figure 10 vertically-elongated L-brackets are attached by nuts and bolts or by screws to lateral members 34I, 36I of frame 12I. The horizontal portions of L-brackets 84 fit into respective ends of C-channels 82. C-channels 82 are drilled to receive vertical struts 80 that are held in place by nuts and washers, not numbered but illustrated in Figure 10. Vertical struts 80 are in turn suspended from the ceiling or other structure high in the room in which unit 10I is located and preferably maintain unit 10I at a level such that the lower surface of unit 10I is above the level of windows and/or eve skylights lighting fixtures supplying light to the room. In this regard it is preferable that the lowest sheet of fabric indicated as 14II in Figure 10 be not only air permeable, antimicrobial and morbidity inducing, preferably comprising a naturally occurring antimicrobial botanical compound, but also be light reflective thereby to effectuate better distribution of light within the room in which unit 10I is installed. Light together with the air treatment provided by unit 10I provides synergistic effects with respect to elimination of SBS syndrome as noted above.

With respect to Figure 11, unit 10G is essentially the same as unit 10I except for presence of the curved lower surface of members 26J. As noted above, such curvature provides greater surface area of air permeable, antimicrobial, morbidity inducing fabric for a given footprint, measured in square area units, of the unit 10J verses 10I. In other respects, unit 10J is the same as unit 10I and the disclosure as set forth above respecting unit 10I is hereby incorporated by reference for unit 10J as illustrated in Figure 11.

Referring to Figure 12, apparatus for preferably passively treating and alleviating SBS to improve indoor air quality is depicted in the form of a vertically upstanding array designated generally 100 that includes a frame designated generally 102 for supporting strips of air permeable, botanically based antimicrobial, morbidity-inducing fabric, where the strips of fabric are designated 14-1, 14-2, 14-3, 14-4 and 14-5. Frame 102 supporting fabric strips 14-1 through 14-5 includes a plurality of upstanding members that are individually designated generally 104. Upstanding members 104 are categorized as first and second upstanding members 106, 108 that are connected front to back by bracing members 110.

Extending laterally between pairs of bracing members 110 and being a part of frame 102 are lateral members 112. In Figure 12, only certain ones of upstanding members 104, first and second upstanding members 106, 108, bracing members 110, and lateral members 112 have been numbered in order to maintain drawing clarity.

Further provided as a portion of frame 102 are cross-braces 114 desirably located at the top of pairs of second upstanding members 108 to increase lateral stability.

A given pair of first and second upstanding members 106, 108 can serve as parts of two adjacent upstanding portions 118 of frame 102 where frame 102 may comprise a number of such adjacent upstanding portions such as five such portions as illustrated in Figure 12. Two such upstanding portions 118 are indicated and so-designated in Figure 12.

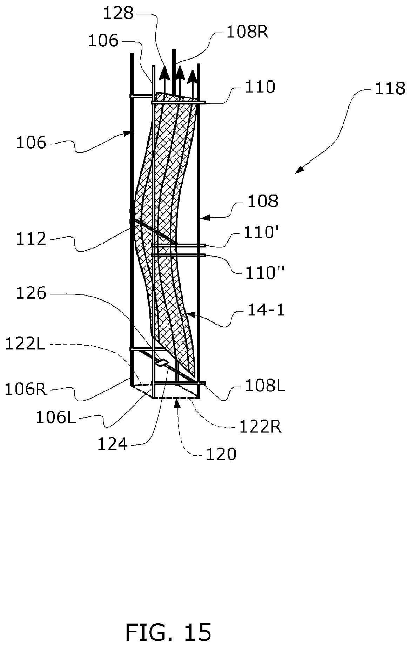

Figure 15 illustrates, in vertically truncated form, a broken segment of one of upstanding portions 118. In Figure 15, vertically upstanding members 106 and 108 are positioned at the corners of an imaginary rectangle, where the rectangle is illustrated in dotted lines and designated 120. The one of first upstanding members 106 at the left hand front side of the rectangle 120 is designated 106L in Figure 15, while the one of first upstanding members 106 at the right hand side of rectangle 120 is designated 106R in Figure 15. Similarly, toe one of second upstanding members 108 at the left hand side of rectangle 120 is designated 108L in Figure 15 and the one of second upstanding members 108 located at the right hand side of rectangle 120 is designated 108R. Upstanding members 106L and 106R are considered to define the front of rectangle 120 where rectangle 120 is provided in this disclosure to clarify the geometry of the structure illustrated in Figure 15.

There may optionally be provided first and second horizontally-oriented support members that are positionable on a floor or other surface to provide vertical support for upstanding portion 118 illustrated in Figure 15; these optionally horizontally-oriented support members would run along the respective dotted lines designated 122L and 122R of rectangle 120 in Figure 15.

As further illustrated in Figure 15, a plurality of vertically-spaced apart parallel bracing members 110 connect respective ones of the upstanding first and second members 106, 108 along respective sides of rectangle 120. Bracing members 100 are preferably provided and oriented in closely vertically-spaced, adjacent pairs as illustrated by parallel bracing members 110', 110" in Figure 15.

A plurality of lateral members 112 extend between and preferably slideably engage the vertically correspondingly positioned pairs 110', 110" of the horizontally-extending parallel bracing members 110. One such lateral member is indicated as 112 in Figure 15. There is further provided a lateral member in the form of a cross-brace 114 at the top of each upstanding portion 118 where the cross-brace 114 is illustrated in Figure 12.

Air permeable, antimicrobial, preferably botanically based, morbidity-inducing fabric, provided in the form of a strip 14-1 as illustrated in Figure 15, is connected at the top of the strip either to an uppermost one of lateral members 112 or to fixed lateral bracing member 114. Fabric strip 14-1 extends downwardly as illustrated in Figure 15 and may be positioned in various configurations by adjusting position of lateral members 112 with fabric strip 14-1 passing on a selected side of a given lateral member 112 thereby to provide the desired configuration for fabric strip 14-1. Specifically, lateral members 112 are moveably positionable along the pairs of parallel bracing members 110, between front and rear with respect to rectangle 120, to cause fabric portions 14-1 connected to the lateral members and extending between the lateral members to conform to selected contours. Desirably, a portion of the selected contour or all of the selected contour may approximate the upper surface of an air foil, in response to positioning of lateral members 112 and in response to air blowing thereagainst or therealong. Positioning of fabric strip 14-1 as the upper surface of an air foil facilitates generation of vortices along the air foil-like surface, thereby contributing to greater air flow through and along fabric strip 14-1, enhancing the antimicrobial, morbidity-inducing effect of fabric 14-1.

Optionally, a fixed horizontal brace illustrated as 124 may be provided at the bottom of Figure 15 with a fan 126 mounted thereon to blow air upwardly against and along fabric strip 14-1 as indicated by arrows 128 at the top of Figure 15.

Referring to Figure 13, the array 100A shown therein is similar to the array 100 illustrated in Figure 12 and is constructed using segments as illustrated in Figure 15. In Figure 13, the upstanding portions 118 illustrated in Figure 15 have been horizontally offset one from another front to back, relative to rectangles 120, thereby to provide a different and possibly more efficient configuration for array 100A. Other than the front to back offset of upstanding portions 118, array 100A in Figure 13 is largely the same as array 100 illustrated in Figure 12, as can be seen by comparing the drawings in which functionally equivalent and substantially corresponding parts have the same number, with the letter "A" used to distinguish parts illustrated in Figure 13 from functionally identical or similar corresponding parts in Figure 12.

With respect to array 100A illustrated in Figure 13, a single first upstanding member 106A could not serve as support for adjacent upstanding portions 118A due to the horizontal offset of the upstanding portions 118A as illustrated in Figure 13. However, a first upstanding member 106A of one upstanding portion 118A could serve as a second or rear upstanding member 108A of an adjacent upstanding portion 118A to horizontally offset as illustrated in Figure 13.

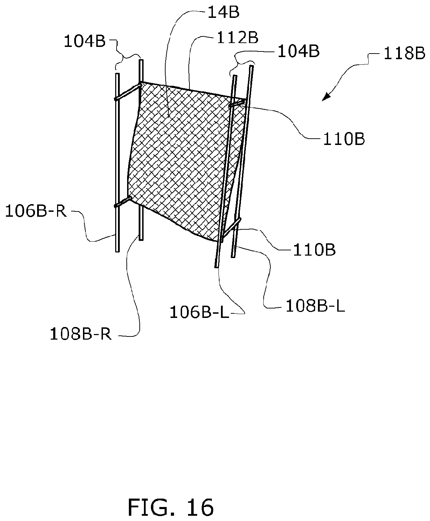

Referring to Figures 14 and 16, Figure 14 illustrates another apparatus for improving indoor air quality in the form of an array 100B where array 100B includes a frame 102B that has vertically upstanding members 104B positioned at the corners of an imaginary rectangle with one edge of the rectangle being considered the front, in much the same manner as illustrated for Figures 12 and 15. Further similarly to Figures 12 and 15, one pair of upstanding members 104B has a first member 106B at the right front of the rectangle and a second member 108B at the right rear of the rectangle, and a second pair of upstanding members 104B having a first member at the left front of the rectangle and second member at the left rear of the rectangle where the members are designated 106B-L, 106B-R, 108B-L and 108B-R, with these designations being most clearly shown in Figure 16. In array 100B illustrated in Figure 14 and in Figure 16, there are further provided a plurality of vertically-spaced apart bracing members 110B connecting respective ones of the upstanding first and second members 106B, 108B of the respective pairs of upstanding members 106B along respective sides of the imaginary rectangle. The imaginary rectangle is not illustrated in Figure 14 nor in Figure 16 to enhance drawing clarity.

As further illustrated in Figure 14, the air permeable, antimicrobial, botanically based, morbidity-inducing fabric is not provided in the form of vertically elongated strips that extend from the top to the bottom of the apparatus 100B. Rather, the air permeable, antimicrobial, botanically based, morbidity-inducing fabric is provided in the form of rectangular sheets 14B where rectangular sheets 14B may be provided as several sheets, one above another, in each upstanding portion 118B of apparatus 100B. Fabric sheets 14B may be secured directly to bracing members 110B desirably by unnumbered rings fitting around bracing members 110B, thereby permitting movement of a fabric sheets 14B between forward upstanding members 106B-L and 106B-R and rear upstanding members 108B-L and 108B-R. Alternatively, lateral members 112B may be provided at either the top or the bottom or both of fabric sheet 14B with lateral members 112B desirably being movable between front and rear along bracing members 110B. With this arrangement, fabric sheets 14B can be adjusted to assume any of a plurality of configurations to take advantage of natural convention in the room in which array 100B is located.

One hundred percent (100%) cotton yarns and 100% cotton woven and knitted fabrics are desirably used to provide the air permeable, botanically based antimicrobial, morbidity inducing fabrics for the apparatus and practice of the methods of the invention. These fabrics are desirably treated using eugenol to impart antimicrobial microorganism morbidity-inducing properties to them.

Specifically, eugenol at 5 or 10 grams per liter may be mixed with polyvinyl alcohol at 5 or 10 grams per liter and 100 grams per liter glyoxal. The material to liquor ratio should be kept at either 1:10 or 1:20 by adding water to make up the difference between the liquor weight and that required by the liquor ratio calculation. The liquor ratio is calculated by weighing the fabric prior to treatment. The solution is desirably applied to the fabric by patting, such as by through the use of a Werner Mathis padder, and the fabric is then dried desirably in a through air oven at 80-85° C for about four minutes. Thereafter, the fabric is cured, desirably in a through air oven, at a temperature ranging from 120-140° C for from about three to about five minutes. With this approach, wet pick up, namely the amount of solution contained in the fabric after padding, amounts to about 65% of the weight of the dried fabric.

Another approach is to utilize instead a solution of eugenol and water, leaving out the polyvinyl alcohol and the glyoxal; the amount of eugenol may also be varied, as may be the liquor ratio and the wet pick up, with a suitable botanically based, antimicrobial, morbidity-inducing fabric resulting.

The fabric may also be made by knitting antimicrobial yarn into a 1x1 rib knit fabric. Plain knit fabric may also be used, but is not as tear-resistant as is rib knit fabric. Accordingly, plain knit fabric may be more difficult to install, whereas the heavier construction of the rib knit fabric eliminates many fabric tearing issues.

Fabrics, whether knit, woven or non-woven, made from different fiber types, including rayon, polyester, nylon and wool, may be used as the antimicrobial fabric.

Natural antimicrobials may be attached to the fabric using different methods. One additional method of attaching a natural antimicrobial botanically based, morbidity-inducing substance is to incorporate clove powder into polypropylene filaments during extrusion of the filaments, by mixing the clove powder with the polypropylene pellets to be extruded. This method incorporates the antimicrobial clove powder into the polypropylene filaments, which may then be knitted or woven into fabrics for use in the apparatus and methods of the invention. Using this method, mixing of the clove powder and the polypropylene pellets must be such that the mix is reasonably uniform. A non-uniform mix results in an undesirable inconsistent blend of clove powder and polypropylene along the length of the resulting polypropylene-clove filaments.

Applying natural botanically-based antimicrobials to other forms of fabrics, such as braided and non-woven, non-knit fabrics is also within the scope of the invention.

The eugenol approach described above for application of natural botanically based, antimicrobials to fabric may also be applied to yarns or fibers, since the fiber does not change its structure during fabric production processes.

Suitable antimicrobials have resulted using fabric knitted from yarns containing recycled fibers. The fiber content of these yarns was about 69% cotton, 29% acrylic, and 2% other materials. These recycled fibers had previously been dyed and had undergone several processing treatments as well as shredding during recycling. Reduction of SBS attained using these fabrics indicates that dyeing does not affect the efficacy of the treatment that produces antimicrobial properties in the fabric.

Knit, woven and non-woven fabrics of different fiber types, such as cotton, rayon, polyester, nylon and wool, can be used in the practice of the invention; these fibers themselves are not antimicrobial. Natural antimicrobials found to be biocidally effective are attached to these fabrics in accordance with the invention.

Typically, a fabric according to the invention may be fabricated using antimicrobial morbidity-inducing synthetic, natural or blended yarns knitted or woven into fabric, for example, a 1x1 ribbed fabric and/or into a 1x1 plain fabric. A desirable heavier construction of ribbed fabric eliminates tearing issues sometimes encountered using plain fabric.

One method for imparting microorganism morbidity-inducing biocidal properties into a fiber in accordance with the invention is to incorporate clove powder, known to be a natural antimicrobial, into polymeric, preferably polypropylene, filaments by extrusion. This may be accomplished by mixing clove powder with polypropylene pellets prior to the mixture being fed through an extruder. The extruder provides as output a filament consisting of the extruded mixture of polypropylene and clove powder. Proper mixing of the clove powder and the pellets prior to the extrusion process is important; if the mix is not uniform, there will not be a consistent blend of clove powder and polypropylene along the length of the extruded filaments. Once the filaments are extruded, those filaments may be woven or knit into fabrics.

Additional aspects of the invention involve combining preferably naturally occurring botanically based antimicrobials with cotton, rayon and other fabrics, by treating the fabric with the botanically based antimicrobial to provide a fabric having biocidal properties. The fabric may be a knit having the ability to be tightly stretched across an SBS unit frame. This aspect of the invention may also be practiced with woven fabrics. Preferable naturally occurring antimicrobials may be applied to other forms of fabric as well, such as braided and non-woven fabrics, and to yarns, fibers and filaments from which these fabrics are made, using the methods of the invention.

The invention further embraces treating fabric to impregnate the fabric with preferably naturally occurring antimicrobials, which is preferable to treating yarns or fibers or filaments except for when extrusion is the method of application of the naturally occurring antimicrobial. When using extrusion, yarn or fiber or filament must be extruded together with the selected natural antimicrobial and then woven or knit into fabric to provide the desired result.

The methods of the invention may be used to impregnate yarns or fibers with naturally occurring antimicrobials, since yarn or fiber does not change its structure during fabric production by knitting or weaving.

The methods of the invention may be used to produce antimicrobial fabrics where the fabrics are initially knitted using yarns containing recycled fibers. The fiber content of these yarns may be in the range of about 69% cotton, about 29% acrylic and about 2% other fiber. Such recycled fibers are preferably previously dyed and preferably undergo processing treatments as well as shedding during the recycling. Dyeing does not affect the efficacy of the fabric treatment that produces the antimicrobial, morbidity-inducing property in the fabric.

A major advantage provided by fabrics treated in accordance with the invention is that these fabrics not only trap microbes but also kill them. This is important since a filter, such as for an air conditioner, a vacuum cleaner and the like, which is not cleaned regularly, can act as an entrapment point for microbes that continue to breed after being trapped there, especially if moisture is present. This is especially important in the case of Bacillus spores of bacteria, which live up to two hundred (200) years.

Some commercially available fabrics are active against some bacteria and are promoted as being "antibacterial", but fabrics in accordance with the invention are also active against mycetes, namely molds, which constitute the dominant airborne microbes in closed rooms. Fabrics in accordance with the invention are more effective relative to commercially available "antibacterial" fabrics, as fabrics in accordance with the invention can be kept in place for at least one month while retaining reactivity and also can be washed at least once without losing their capacity for inducing morbidity in microorganisms.

Since fabrics in accordance with the invention are preferably made by treatment with natural biocides, a consumer can wash the fabrics in accordance with the invention in a home washing machine. Also, since fabrics in accordance with the invention are made by treatment with naturally occurring biocides, fabrics may be discarded without producing a health hazard. Fabrics in accordance with the invention are preferably biodegradable.

Among the naturally occurring antimicrobial, morbidity-inducing materials that may be used in the course of practice of the invention in treating fabric are echinacea, calendula, aloe vera, turmeric, chamomile, cloves and eugenol, with the latter preferably coming from the clove plant. Cloves and eugenol from cloves are the most preferable, and may preferably be used with natural fabrics such as cotton and silk; with semi-artificial fabrics such as cotton-polyester and cotton-rayon; and with artificial fabrics such as viscose and rayon. The methods of the invention couple these naturally occurring microbicidal materials to the fabrics; the resulting fabrics retain their new microbicidal properties, even following substantial air exposure and washing.

*In vitro* studies comparing the fabrics of the invention with commercial antibacterial fabric demonstrate that fabrics treated according to the invention are at least as good at killing bacteria as commercially available antibacterial fabric and, in addition, fabrics treated according to the invention kills the molds.

### Example 1

Eugenol, in the form of crushed cloves, was mixed with Pro-Fax MI40 polypropylene pellets obtained from Himont, Inc. The pellets had a diameter of approximately 3mm. Mixing was done manually using a stirring rod and beaker. The mixture was then extruded using an Atlas Laboratory mixing extruder at a temperature of about 200° C. The filaments were air cooled, collected, and found to exhibit antimicrobial morbidity-inducing properties.

### Example 2

One hundred percent (100%) cotton yarn and 100% cotton woven and knitted fabric were treated using eugenol derived from cloves to impart microorganism morbidity-inducing properties to the yarn and fabrics. Specifically, the eugenol at 5 grams per liter was mixed with polyvinyl alcohol at 5 grams per liter and 100 grams per liter glyoxal. The material to liquor ratio was kept at 1:10 by adding water to make up the difference between the liquor weight and that required by the liquor ratio calculation. The liquor ratio was calculated by weighing the fabric prior to treatment. The solution was applied to the yarn and to the fabric using a Werner Matthis padder. The yarn and fabric were then dried in a through air oven at between about 80° and about 85° C for four minutes. The yarn and fabric were then cured in the through air oven at a temperature ranging from about 120° to about 140° C for from about three to about five minutes. Wet pick up, namely the amount of solution contained in the fabric after padding, was about 65% of the weight of the dried yarn and fabric. The yarns and fabrics were found to have antimicrobial morbidity inducing properties.

### Example 3

One hundred percent (100%) cotton yarn and 100% cotton woven and knitted fabrics were treated using eugenol derived from cloves to impart microorganism morbidity-inducing properties in the yarns and fabrics. Eugenol at 10 grams per liter was mixed with polyvinyl alcohol at 10 grams per liter and 100 grams per liter glyoxal. The material to liquor ratio was kept at 1:20 by adding water to make up the difference between the liquor weight and that required by the liquor ratio calculation. The liquor ratio was calculated by weighing the yarn and the fabric prior to treatment. The solution was applied to the yarn and to the fabric through the use of a Werner Matthis padder. The yarn and the fabric were then dried in a through air oven at between about 80° and about 85° C for four minutes. The yarn and the fabric were then cured in the through air oven at a temperature ranging from about 120° to about 140° C for from about three to about five minutes. Wet pick up, namely the amount of solution contained in the fabric after padding, was about 65% of the weight of the dried fabric. The yarn and fabric were found to have antimicrobial morbidity-inducing properties.

Regarding Examples 2 and 3, it is also within the scope of the invention to utilize a solution of eugenol and water, leaving out the polyvinyl alcohol and the glyoxal. Likewise, it is within the scope of the invention to vary the amount of eugenol, as well as to vary the liquor ratio and the wet pick up.

In summary and most desirably, one hundred percent (100%) cotton yarns and 100% cotton woven and knitted fabrics may be used to provide the air permeable, botanically based antimicrobial, morbidity inducing fabrics of the invention. These fabrics are preferably treated using eugenol to impart antimicrobial microorganism morbidity-inducing properties to them; other naturally occurring biocidal botanicals may also be used.

Desirably, eugenol at 5 or 10 grams per liter may be mixed with polyvinyl alcohol at 5 or 10 grams per liter and 100 grams per liter glyoxal. The material to liquor ratio should be kept at either 1:10 or 1:20 by adding water to make up the difference between the liquor weight and that required by the liquor ratio calculation. The liquor ratio is calculated by weighing the fabric prior to treatment. The solution is desirably applied to the fabric by patting, such as by through the use of a Werner Mathis padder, and the fabric is then dried desirably in a through air oven at 80-85° C for about four minutes. Thereafter, the fabric is cured, desirably in a through air oven, at a temperature ranging from 120-140° C for from about three to about five minutes. With this approach, wet pick up, namely the amount of solution contained in the fabric after padding, amounts to about 65% of the weight of the dried fabric.

Another approach is to utilize instead a solution of eugenol and water, leaving out the polyvinyl alcohol and the glyoxal; the amount of eugenol may also be varied, as may be the liquor ratio and the wet pick up, with a suitable botanically based, antimicrobial, morbidity-inducing fabric resulting.

The fabric may also be made by knitting antimicrobial yarn into a 1x1 rib knit fabric. Plain knit fabric may also be used, but is not as tear-resistant as is rib knit fabric. Accordingly, plain knit fabric may be more difficult to install on an SBS treatment unit, whereas the heavier construction of the rib knit fabric eliminates many fabric tearing issues.

Fabrics, whether knit, woven or non-woven, made from different fiber types, including rayon, polyester, nylon and wool, may be used to make antimicrobial fabric in accordance with the invention.

Natural botanical antimicrobials may be attached to the fabric using different methods. One additional method, similar to that described above as Example 1, of attaching a natural antimicrobial botanically based, morbidity-inducing substance is to incorporate clove powder into polypropylene filaments during extrusion of the filaments, by mixing the clove powder with the polypropylene pellets to be extruded. This method incorporates the antimicrobial clove powder into the polypropylene filaments, which may then be knitted or woven into fabrics for use in the apparatus and methods of the invention. Using this method, mixing of the clove powder and the polypropylene pellets must be such that the mix is reasonably uniform. A non-uniform mix results in an undesirable inconsistent blend of clove powder and polypropylene along the length of the resulting polypropylene-clove filaments. The same extrusion process may be utilized with corn gluten meal being used in place of clove powder.

Applying natural botanically-based antimicrobials to other forms of fabrics, such as braided and non-woven, non-knit fabrics is also within the scope of the invention.

The eugenol approach described above for application of natural botanically based, antimicrobials to fabric may also be applied to yarns or fibers, since the fiber does not change its structure during fabric production processes.

Suitable antimicrobials result using fabric knitted from yarns containing recycled fibers. The recycled fibers may have been previously dyed and may have undergone several processing treatments as well as shredding during recycling. Dyeing does not affect the efficacy of the treatment that produces antimicrobial properties in the fabric.

As set forth herein in practice of the preferred embodiment of the invention, eugenol is the preferable antimicrobial, naturally occurring biocide to be used in treating fabrics in accordance with the invention. However, eugenol is not the only antimicrobial, naturally occurring biocide that may be used in the practice of the invention. Other suitable antimicrobial, naturally occurring biocides are cloves, calendula, aloe vera and chamomile.

While eugenol is sometimes called "clove oil" because it is the active element in cloves, eugenol is an allyl chain-substituted guaiacol (2-methoxyphenol). Oil extracted from cloves, which is sometimes called "eugenol" herein, has been found to be much more effective as antimicrobial biocide than ordinary commercially available eugenol. Specifically, it takes about five times as much commercially available eugenol to produce the same antimicrobial biocidal effect as clove oil extracted directly from cloves. Calendula and chamomile are required to be used at at least a 5% concentration in solution to be effective. Clove oil at a 0.1% concentration is an effective antimicrobial biocidal, as is eugenol at a 0.5% concentration. Aloe vera, if used, must be used at a 5% concentration.

### The present disclosure also provides the following numbered embodiments:

1) A modular unit for improving indoor air quality, comprising:
   a) a frame surrounding an open interior and defining the outer periphery of the unit;
   b) air permeable, antimicrobial, morbidity-inducing fabric, comprising a naturally occurring antimicrobial botanical compound, secured about the frame periphery on a first side of the frame and covering the open interior on a first side of the frame;
   c) an air impermeable member secured about and covering the frame periphery on a remaining side of the frame;
   d) at least one aperture formed in the frame and being adapted to house a fan therein;
   e) a fan housed in the aperture for blowing air from outside the frame into the frame interior for subsequent passage of air blown into the frame interior outwardly
   f) through the fabric.
2) The modular unit for improving indoor air quality of embodiment 1, further comprising:
   a) the frame including a pair of parallel spaced apart lateral members connected by at least one tensioning cable and at least one aperture opening into the open center;
   b) the frame periphery on a remaining side of the frame being adapted to fit against a wall;
   c) the cable(s) being adapted to connect to one or more cable gripping devices mounted in a wall so upon connection to the cable gripping devices the cable pulls the remaining side of the frame against the wall thereby closing the unit interior so that air may enter the unit interior only by action of the fan.
3) The modular unit for improving indoor air quality of embodiment 2, further comprising:
   a) the unit being mountable against a vertical wall by a portion of the frame hanging on at least one member protruding from the wall, with a second side of the frame being flush with the wall so that air blown into the open interior of the frame by the fan escapes from the frame interior by passing through the fabric.
4) A modular unit for improving indoor air quality, comprising:
   a) a horizontal frame surrounding an open interior and defining a portion of the outer periphery of a filter portion of the unit having an outwardly facing upwardly elongated surface;
   b) air permeable, antimicrobial, morbidity-inducing fabric comprising a naturally occurring antimicrobial botanical compound secured around the frame periphery on a downwardly facing side of the frame and covering the open interior on the downwardly facing side of the frame;
   c) porous light-reflective fabric secured about the frame periphery on the upwardly facing side of the frame and covering the frame periphery on the upwardly facing side of the frame;
   d) at least one fan mounted in the frame for blowing air from outside the frame into the frame interior, for subsequent passage of air blown into the frame interior, outwardly through the air permeable fabric.
   e) an exterior portion of the frame outer periphery being planar for facingly contacting a portion of a window;
   f) at least one member extending upwardly from the frame for upwardly supporting the frame about a pivotal connection at a position remote from the planar portion of the frame to permit weight of the frame to urge the frame against the portion of the window and maintain the frame in a horizontal plane;
   g) at least one solar cell positionable to receive solar energy through the window, connected to and powering the fan.
5) Apparatus for combating sick building syndrome, comprising:
   a) fabric comprising at least one naturally occurring substance exhibiting microorganism morbidity inducing properties; and
   b) a frame adapted for affixation of the fabric thereacross; and
   c) a fan for blowing air through a portion of the fabric affixed across the frame.
6) Apparatus of embodiment 5 wherein the naturally occurring anti-bacterial substance is botanically based.
7) Apparatus of embodiment 6 wherein the naturally occurring substance exhibiting microorganism morbidity inducing properties is clove oil.
8) Apparatus of embodiment 6 wherein the naturally occurring substance exhibiting microorganism morbidity inducing properties is eugenol.
9) A method for combating sick building syndrome, comprising:
   a) providing a fabric comprising at least one naturally occurring substance exhibiting microorganism morbidity inducing properties, affixed across a frame; and
   b) blowing air through a portion of the fabric affixed across the frame.
10) The method of embodiment 9 wherein the naturally occurring substance exhibiting microorganism morbidity inducing properties is clove oil.
11) The method of embodiment 9 wherein the naturally occurring substance exhibiting microorganism morbidity inducing properties is eugenol.
12) Apparatus for improving indoor air quality, comprising:
   a) vertically upstanding members positioned at the corners of an imaginary rectangle, one edge of the rectangle being considered the front;
   b) one pair of members having a first member at the right front of the rectangle and the second member at the right rear of the rectangle and a second pair of members having a first member at the left front of the rectangle and a second member at the left rear of the rectangle;
   c) a plurality of vertically spaced apart bracing members connecting respective ones of the upstanding first and second members of the respective pairs of upstanding members along respective sides of the rectangle; and
   d) air permeable, botanically based antimicrobial, morbidity-inducing fabric connected to and extending between vertically adjacent pairs of lateral members.
13) Apparatus of embodiment 12 further comprising:
   a) lateral members extending between vertically correspondingly positioned pairs of the horizontally extending parallel bracing members;
   b) the lateral members being movably positionable along the parallel bracing members, between front and rear, to cause the fabric portions connected thereto and extending therebetween to conform to selected contours, a portion of or all of which may approximate the upper surface of an airfoil, in response to air blowing thereagainst.
14) A method for treating fabric to make it antimicrobial, comprising:
   a) weighing a portion of fabric to be treated;
   b) selecting a desired ratio of fabric weight to weight of solution of naturally occurring active antimicrobial material;
   c) blending the active naturally occurring antimicrobial material and liquid in a ratio of 5 to 10 grams of active natural antimicrobial material per liter of liquid;
   d) adding more liquid to the blend in an amount that the resulting solution weighs between about 10 to about 20 times that of the fabric portion to be treated;
   e) applying the solution to the fabric;
   f) drying the wetted fabric at a temperature of between about 80° and about 85°C; and
   g) curing the dried fabric at a temperature of between about 120° and about 140°C for from about three to about five minutes.
15) The method of embodiment 14 wherein the liquid is water.
16) The method of embodiment 14 wherein the active natural antimicrobial material is selected from the group comprising eugenol and clove powder.
17) The method of embodiment 14 wherein the solution is applied to the fabric by contacting the fabric with a pad impregnated with the solution.
18) The method of embodiment 14 wherein drying the wetted fabric is performed in a through air oven.
19) The method of embodiment 14 wherein curing the dried fabric is performed in a through air oven.
20) A method for making an antimicrobial fabric, comprising the steps of:
   a) providing a mixture of pellets of at least one polymer material that can be extruded into weavable or knittable filament;
   b) blending the pellet mixture with a naturally occurring antimicrobial substance;
   c) extruding the resulting blend into filaments;
   d) weaving or knitting the filaments into fabric.

## Claims

1. Apparatus for improving indoor air quality, comprising:
a) vertically upstanding members positioned at the corners of an imaginary rectangle, one edge of the rectangle being considered the front;
b) one pair of members having a first member at the right front of the rectangle and the second member at the right rear of the rectangle and a second pair of members having a first member at the left front of the rectangle and a second member at the left rear of the rectangle;
c) a plurality of vertically spaced apart bracing members connecting respective ones of the upstanding first and second members of the respective pairs of upstanding members along respective sides of the rectangle; and
d) air permeable, botanically based antimicrobial, morbidity-inducing fabric connected to and extending between vertically adjacent pairs of lateral members.

2. Apparatus of claim 1 further comprising:
a) lateral members extending between vertically correspondingly positioned pairs of the horizontally extending parallel bracing members;
b) the lateral members being movably positionable along the parallel bracing members, between front and rear, to cause the fabric portions connected thereto and extending therebetween to conform to selected contours, a portion of or all of which may approximate the upper surface of an airfoil, in response to air blowing thereagainst.

3. A modular unit for improving indoor air quality, comprising:
a) a horizontal frame surrounding an open interior and defining a portion of the outer periphery of a filter portion of the unit having an outwardly facing upwardly elongated surface;
b) air permeable, antimicrobial, morbidity-inducing fabric comprising a naturally occurring antimicrobial botanical compound secured around the frame periphery on a downwardly facing side of the frame and covering the open interior on the downwardly facing side of the frame;
c) porous light-reflective fabric secured about the frame periphery on the upwardly facing side of the frame and covering the frame periphery on the upwardly facing side of the frame;
d) at least one fan mounted in the frame for blowing air from outside the frame into the frame interior, for subsequent passage of air blown into the frame interior, outwardly through the air permeable fabric.
e) an exterior portion of the frame outer periphery being planar for facingly contacting a portion of a window;
f) at least one member extending upwardly from the frame for upwardly supporting the frame about a pivotal connection at a position remote from the planar portion of the frame to permit weight of the frame to urge the frame against the portion of the window and maintain the frame in a horizontal plane;
g) at least one solar cell positionable to receive solar energy through the window, connected to and powering the fan.

4. Apparatus for combating sick building syndrome, comprising:
a) fabric comprising at least one naturally occurring substance exhibiting microorganism morbidity inducing properties; and
b) a frame adapted for affixation of the fabric thereacross; and
c) a fan for blowing air through a portion of the fabric affixed across the frame.

5. Apparatus of claim 4 wherein the naturally occurring anti-bacterial substance is botanically based.

6. Apparatus of claim 5 wherein the naturally occurring substance exhibiting microorganism morbidity inducing properties is clove oil.

7. Apparatus of claim 5 wherein the naturally occurring substance exhibiting microorganism morbidity inducing properties is eugenol.

8. A method for combating sick building syndrome, comprising:
a) providing a fabric comprising at least one naturally occurring substance exhibiting microorganism morbidity inducing properties, affixed across a frame; and
b) blowing air through a portion of the fabric affixed across the frame.

9. The method of claim 8 wherein the naturally occurring substance exhibiting microorganism morbidity inducing properties is clove oil.

10. The method of claim 8 wherein the naturally occurring substance exhibiting microorganism morbidity inducing properties is eugenol.
a)

11. A method for treating fabric to make it antimicrobial, comprising:
a) weighing a portion of fabric to be treated;
b) selecting a desired ratio of fabric weight to weight of solution of naturally occurring active antimicrobial material;
c) blending the active naturally occurring antimicrobial material and liquid in a ratio of 5 to 10 grams of active natural antimicrobial material per liter of liquid;
d) adding more liquid to the blend in an amount that the resulting solution weighs between about 10 to about 20 times that of the fabric portion to be treated;
e) applying the solution to the fabric;
f) drying the wetted fabric at a temperature of between about 80° and about 85°C; and
g) curing the dried fabric at a temperature of between about 120° and about 140°C for from about three to about five minutes.

12. The method of claim 11 wherein the liquid is water, or wherein the active natural antimicrobial material is selected from the group comprising eugenol and clove powder, or wherein the solution is applied to the fabric by contacting the fabric with a pad impregnated with the solution.

13. The method of claim 11 wherein drying the wetted fabric is performed in a through air oven.

14. The method of claim 11 wherein curing the dried fabric is performed in a through air oven.

15. A method for making an antimicrobial fabric, comprising the steps of:
a) providing a mixture of pellets of at least one polymer material that can be extruded into weavable or knittable filament;
b) blending the pellet mixture with a naturally occurring antimicrobial substance;
c) extruding the resulting blend into filaments;
d) weaving or knitting the filaments into fabric.
